Europäisches Patentamt

European Patent Office

Office européen des brevets

(19)

(11) **EP 0 646 215 B1**

(12) **EUROPEAN PATENT SPECIFICATION**

(45) Date of publication and mention
of the grant of the patent:
**20.10.1999 Bulletin 1999/42**

(51) Int Cl.⁶: **E21B 49/10**, E21B 47/06,
E21B 47/12, F04B 53/00,
G01N 33/24

(21) Application number: **93916625.2**

(22) Date of filing: **18.06.1993**

(86) International application number:
**PCT/US93/05875**

(87) International publication number:
**WO 94/00671 (06.01.1994 Gazette 1994/02)**

(54) **METHOD AND APPARATUS FOR PRESSURE, VOLUME, AND TEMPERATURE MEASUREMENT AND CHARACTERIZATION OF SUBSURFACE FORMATIONS**

VERFAHREN UND VORRICHTUNG FÜR DRUCK-, VOLUMEN UND TEMPERATURMESSUNGEN UND ZUR CHARAKTERISIERUNG VON UNTERTÄGIGEN FORMATIONEN

PROCEDE ET APPAREIL DE MESURE DE TEMPERATURE, DE PRESSION ET DE VOLUME, ET DE CARACTERISATION DE FORMATIONS SOUTERRAINES

(84) Designated Contracting States:
**FR GB NL**

(30) Priority: **19.06.1992 US 903088**
**16.04.1993 US 48814**

(43) Date of publication of application:
**05.04.1995 Bulletin 1995/14**

(73) Proprietor: **WESTERN ATLAS INTERNATIONAL, INC.**
**Houston, TX 77042-3115 (US)**

(72) Inventors:
• **LEDER, John, T.**
**Bellaire, TX 77401 (US)**
• **MICHAELS, John, M.**
**Houston, TX 77042 (US)**
• **SHWE, Than**
**Houston, TX 77099 (US)**
• **ANDERSON, Crawford, A.**
**Houston, TX 77077 (US)**

(74) Representative: **Godsill, John Kenneth et al**
**Haseltine Lake & Co.,**
**Imperial House,**
**15-19 Kingsway**
**London WC2B 6UD (GB)**

(56) References cited:
| | |
|---|---|
| US-A- 3 209 588 | US-A- 3 492 946 |
| US-A- 3 611 799 | US-A- 3 657 925 |
| US-A- 3 813 936 | US-A- 4 339 948 |
| US-A- 4 434 653 | US-A- 4 535 843 |
| US-A- 4 745 802 | US-A- 5 051 074 |
| US-A- 5 065 619 | |

**Description**

This invention relates generally to a method and apparatus for subsurface formation testing, and more particularly concerns methods and apparatus for providing real time simultaneous measurements of the response to changes in pressure, volume and/or temperature of fluid samples from subsurface earth formations traversed by a wellbore for conducting in situ tests for determination of factors influencing potential formation productivity and for acquiring connate fluid samples in sample tanks under conditions of controlled pressure for subsequent laboratory analyses. Such in situ tests include determination of fluid bubble point pressure at formation depth and under formation conditions and determination of fluid compressibility and use of the same for reservoir characterization by means of bubble point pressure and fluid compressibility profile plots.

The sampling of fluids contained in subsurface earth formations provides a method of testing formation zones of possible interest by recovering a sample of any formation fluids present for later analysis in a laboratory environment while causing a minimum of damage to the tested formations. The formation sample is essentially a point test of the possible productivity of subsurface earth formations. Additionally, a continuous record of the sequence of events during the test is made at the surface. From this record, valuable formation pressure and permeability data as well as data determinative of fluid compressibility and relative viscosity can be obtained for formation reservoir analysis.

Early formation fluid sampling instruments such as the one described in U.S. Patent No. 2,674,313 were not fully successful as a commercial service because they were limited to a single test on each trip into the borehole. Later instruments were suitable for multiple testing; however, the success of these testers depended to some extent on the characteristics of the particular formations to be tested. For example, where earth formations were unconsolidated, a different sampling apparatus was required than in the case of consolidated formations.

Down-hole multi-tester instruments have been developed with extensible sampling probes for engaging the borehole wall at the formation of interest for withdrawing fluid samples therefrom and measuring pressure. In downhole instruments of this nature it is typical to provide an internal draw-down piston which is reciprocated hydraulically or electrically to increase the internal volume of a fluid receiving chamber within the instrument after engaging the borehole wall. This action reduces the pressure at the instrument formation interface causing fluid to flow from the formation into the fluid receiving chamber of the tool. Heretofore, the pistons accomplish suction activity only while moving in one direction. On the return stroke the piston simply discharges the formation fluid sample through the same opening through which it was drawn and thus provides no pumping activity. Additionally, unidirectional piston pumping systems of this nature are capable of moving the fluid being pumped in only one direction and thus causes the sampling system to be relatively slow in operation.

Early down-hole multi-tester instruments were not provided with a capacity for substantially continuous pumping of formation fluid. Even large capacity tools have heretofore been limited to a maximum draw-down collection capability of only about 1000 cc and they have not heretofore had the capability of selectively pumping various fluids to and from the formation, to and from the borehole, from the borehole to the formation, or from the formation to the borehole. U.S. Patent 4,513,612 describes a Multiple Flow Rate Formation Testing Device and Method which allows the relatively small volume draw-down volume to be discharged into the wellbore or to be forced back into the formation. The use of "passive" valves as taught in this method precludes reverse flow. This method does provide for limited or one shot reverse flow much like a hypodermic needle but transferring large volumes of fluid between two reservoirs in a near continuous manner is not achievable with this method. It is desirable, therefore, to provide a down-hole fluid sampling tool with enhanced pumping capability with an unlimited capacity for discharge of formation fluid into the wellbore and with the capability to achieve bi-directional fluid pumping to enable a reverse flow activity that permits fluid to be transferred to or from a formation. It is also desirable to provide a down-hole testing instrument having the capability of selectively pumping differing fluids such as formation fluid, known oils, known water, known mixtures of oil and water, known gas-liquid mixtures, and/or completion fluid to thereby permit in situ determination of formation permeability, relative permeability and relative viscosity and to verify the effect of a selected formation treatment fluid on the producibility of connate fluid present in the formation.

In all cases known heretofore, down-hole multi-test sampling apparatus incorporates a fluid circuit for the sampling system which requires the connate fluid extracted from the formation, together with any foreign matter such as fine sand, rocks, mud-cake, etc. encountered by the sampling probe, to be drawn into a relatively small volume chamber and which is discharged into the borehole when the tool is closed as in U.S. Patent 4,416,152. Before closing, a sample can be allowed to flow into a sample tank through a separate but parallel circuit. Other methods provide for the sample to be collected through the same fluid circuit.

U.S. Patent 3,813,936 describes a "valve member 55" in column 11, lines 10-25 which forces trapped wellbore fluids in a "reverse flow" through a screen member as the "valve member 55" is retracted. This limited volume reverse flow is intended to clean the screen member and is not comparable to bi-directional flow described in this disclosure because of the limited volume.

Mud filtrate is forced into the formation during the drilling process. This filtrate must be flushed out of the formation before a true, uncontaminated sample of the connate fluid can be collected. Prior art sampling devices have a first sample tank to collect filtrate and a second to collect connate fluid. The problem with this procedure is that the volume of filtrate to be removed is not known. For this reason it is desirable to pump formation fluid that is contaminated with filtrate from the formation until uncontaminated connate fluid can be identified and produced. Conventional down-hole testing instruments do not have an unlimited fluid pumping capability and therefore cannot ensure complete flushing of the filtrate contaminant prior to sampling.

Estimates of formation permeability are routinely made from the pressure change produced with one or more draw-down piston. These analyses require that the viscosity of the fluid flowing during pumping be known. This is best achieved by injecting a fluid of known viscosity from the tool into the formation and comparing its viscosity with recovered treated formation fluid.

A reversible pump direction will also allow the fluid to be injected from the tool or borehole into the formation. For example, treatment fluid stored within an internal tank or compartment of the instrument or drawn from the wellbore may be injected into the formation. After injection, additional draw-downs and/or sampling may take place to determine the effect of the treatment or completion fluid on the producibility of the formation. Early formation sampling instruments have not been provided with features to determine the optimum sampling pressures. The present invention also provides a positive method for overcoming differential sticking of the packer.

Determining whether or not thin bed formations are truly connected is a significant concern when completing an oil/gas well for production. This is presently done with pressure gradient plots; however, when the formations are near the same depth, the use of pressure gradient plots may not be conclusive.

An alternative to increasing the accuracy of depth measurement is to examine some other physical characteristic which can also be correlated to common or separate formations. The development of downhole pressure, volume and temperature measurement through employment of apparatus and methods as set forth herein can effectively provide these characteristics.

Bubble point pressure is a physical property of formation fluid which is defined as the pressure at which a gas begins to be liberated from a liquid/gas mixture and a gas bubble begins to form in the fluid at a constant temperature. Bubble point pressure is determined by confining fluid in a known volume and by observing pressure changes as the volume of the fluid is changed. A plot of volume in comparison with pressure will indicate the fluid phase change from one phase (liquid) into two phases (liquid and gas). The intersection of two best fit "volume" and "pressure" lines of the plot indicates the bubble point pressure for the sample fluid.

Once a petroleum field is discovered, it is essential to know details about the fluid content and the geological constraints of the reservoir in order to maximize recovery of the petroleum products therefrom. Most known oil and gas reservoirs are stratified and multilayered. Some producible petroleum reservoirs may be 300 m (1000 feet) thick and others as thin as 0.3 m (1 foot). The different layers of the reservoir may contain the same type of fluid or may contain fluids of different origins, driven by the same pressure source or driven by different pressure sources. Determining which of these conditions is true before completing a well for production is very significant in efficient recovery of hydrocarbons from the formation or formations that are intersected by the well bore.

Bubble point pressure data has been used for fluid composition analysis using the recombined fluid samples. Such data has not been used heretofore for purposes of reservoir characterization, principally because of the inability to obtain representative fluid samples in quantity, quality and in a timely manner as required for reservoir interpretation. It is therefore desirable to provide a downhole PVT multitester instrument and method for conducting bubble point fluid analysis and determination of fluid compressibility at formation depth and to employ the results thereof for the purpose of reservoir characterization.

The object of the present invention is to overcome the deficiencies of the prior art by providing method and apparatus for achieving in situ pressure, volume and temperature (PVT) measurement and bubble point pressure and fluid compressibility analysis. These features are accomplished through utilization of a PVT multitester instrument having a double-acting, bi-directional fluid control system incorporating a double-acting bi-directional piston pump capable of achieving pumping activity in each direction of its linear stroke and capable, through its reciprocating pump stroke and valving activity, of achieving bi-directional fluid flow. The downhole PVT instrument is also capable of selectively discharging acquired connate fluid into the wellbore or into sample containing vessels or pumping fluid from the wellbore or a sample containing vessel into the formation.

For bubble point and fluid compressibility analysis at least one of the pumping chambers of the PVT multitester instrument is designated as a bubble point and fluid compressibility test chamber for valve controlled confinement of a known volume of the formation fluid. By then accurately sensing the temperature and pressure of the trapped volume of fluid, such as by means of precision temperature and pressure sensors in communication with the test chamber, and by selectively changing the volume of the test chamber by piston movement and accurately detecting the volumetric test chamber change, such as by means of a precision controlled linear potentiometer, electronic signals representing fluid temperature pressure and volumetric change can be readily acquired and utilized for bubble point pressure profile plots for use in reservoir characterization.

It is a principle feature of the present invention to provide a novel method for accomplishing down-hole pressure, volume and temperature measurement of connate fluid being extracted from a formation of interest and for pressurizing or depressurizing the connate fluid sample for the construction of PVT relationship curves which can be used to determine the type of reservoir fluid prior to taking a sample.

It is also a feature of this invention to provide a novel method for pressure volume and temperature measurement of connate fluid present in a subsurface formation of interest wherein the bubble point pressure of fluid is determined in order to set up the optimum sampling pressure for collecting a sample representative of the reservoir condition.

It is another feature of this invention to provide a novel method and apparatus for accomplishing down-hole testing of a subsurface formation to enable the identification of the fluid prior to collection of samples, to determine down-hole bubble point pressure for optimum sampling conditions, to determine fluid properties for pressure transient analysis, and to accomplish acquisition of down-hole pressure volume and temperature data to check the validity of samples at the surface, either on location or at reservoir fluid laboratories.

It is also a feature of the present invention to provide a downhole PVT multitester instrument that has defined therein a test chamber and signal acquisition system for bubble point pressure and fluid compressibility analysis at formation depth to provide for efficient bubble point and fluid compressibility profile plots for use in reservoir characterization.

It is another important feature of this invention to provide controlled draw-down pressure or sample flowing pressure to improve pressure transient analysis of a draw-down and build-up test.

It is another important feature of this invention to provide for control of the injection pressure of fluid being injected into the formation to improve pressure transient analysis of an injection test.

It is another important feature of this invention to accomplish in situ pressure transient analysis in the down-hole environment.

It is another important feature of this invention to determine fracturing pressure of the formation, which is critical data for formation stimulation.

It is another important feature of this invention to provide novel down-hole testing apparatus incorporating a double-acting bi-directional pump which is contained within a draw-down module having a pump through capability and wherein the pump speed can be controlled thus allowing a controlled rate for pressure drop, total volume or rate so as to adapt the test being conducted to the formation characteristics.

It is another feature of this invention to provide novel apparatus for down-hole formation testing which incorporates a pump and valving mechanism enabling the selective pumping of the fluid from the formation to the borehole so as to accomplish removal of all filtrate from the formation before sampling or taking other formation data such as pressure, volume and temperature (PVT), resistivity, compressibility, bubble point, relative viscosity, etc.

It is an even further feature of this invention to provide a novel down-hole testing apparatus incorporating a double-acting bi-directional pump mechanism capable of reversing its pumping direction to allow fluid, such as completion fluid, to be injected from the instrument or borehole into the formation and to accomplish draw-down from the formation for sampling to determine the effect of the fluid being injected into the formation.

It is another feature of this invention to provide a novel formation testing instrument having a down-hole draw-down pump-through capability and having a double-acting bi-directional pump and valving arrangement capable of accomplishing selective pumping of an unlimited volume of formation fluid from the formation to the wellbore or to the collection tanks to clean or flush away any debris such as sand, rocks, filtrate, etc. in the formation surrounding the wellbore or at the interface of the sampling probe with the wellbore wall to ensure the taking of a clean, uncontaminated sample for testing.

It is an even further feature of this invention to provide a novel formation testing instrument having a down-hole draw-down double-acting bi-directional pump mechanism which allows fluid to be injected from the tool or borehole into the formation to determine the effect of the completion fluid on the formation and its constituents.

According to one aspect of the present invention as defined in claim 1, there is provided a method for testing a subsurface earth formation having a wellbore defined therein and containing formation fluid, comprising:

(a) positioning within said wellbore a formation testing instrument having a sampling probe and having a bi-directional piston pump therein being in pumping communication with said sampling probe for selective pumping into and from said subsurface earth formation and having at least one internal sample tank;

(b) projecting said sampling probe from said formation testing instrument into sampling communication with said wellbore at said subsurface earth formation;

(c) with said bi-directional piston pump forcing a quantity of testing fluid through said sampling probe and into said subsurface earth formation; and

(d) with said bi-directional piston pump, withdrawing a quantity of said formation fluid from said subsurface earth formation through said sampling probe and into said formation testing instrument.

According to a second aspect of the present invention as defined in claim 19, there is provided a method for flushing debris from a substrate earth formation having a wellbore defined therein and for acquiring uncontaminated samples of formation fluid for testing thereof, said formation fluid having a bubble point pressure, said method further comprising:

(a) positioning within said wellbore a formation testing instrument having a sampling probe and having an on-board fluid tank and having a bi-directional piston pump in selective pumping communication with said sampling probe and said onboard fluid tank;
(b) projecting said sampling probe from said formation testing instrument into sampling communication with said subsurface earth formation;
(c) pumping formation fluid from said subsurface earth formation through said formation testing instrument and into said wellbore until uncontaminated formation fluid is recovered; and
(d) pumping a quantity of said uncontaminated formation fluid into said onboard fluid tank.

According to a third aspect of the present invention as defined in claim 23, there is provided apparatus for conducting pressure, volume and temperature tests of a connate fluid sample from a subsurface earth formation having a wellbore defined therein and for obtaining samples of connate fluid contained within said subsurface earth formation, comprising:

(a) an instrument body structure for positioning at a selected formation depth within said wellbore and having a fluid tank therein containing testing fluid;
(b) a sample probe being laterally extensible from said instrument body structure for fluid sampling engagement with said subsurface earth formation, said sample probe defining a fluid sampling passage for admitting said connate fluid sample from said subsurface earth formation to said instrument and for transferring fluid from said instrument to said subsurface earth formation;
(c) a bi-directional piston pump being located within said instrument body structure and having a pump cylinder and a piston being movable within said pump cylinder; and
(d) means selectively controlling the pumping direction of said piston pump mechanism for selective pumping of said testing fluid through said sampling probe into said surface earth formation and pumping of formation fluid from said subsurface earth formation through said sampling probe and into said body structure.

According to a fourth aspect of the present invention as defined in claim 43, there is provided a down-hole formation testing instrument comprising:

(a) an instrument body adapted for positioning at formation depth within a wellbore and defining a pump chamber a fluid sampling and pumping passage in communication with said pump chamber;
(b) a sample probe cooperatively arranged on and laterally extensible from said instrument body structure for fluid sampling engagement with a selected subsurface earth formation, said sample probe defining a fluid sample passage for admitting connate fluid from said subsurface earth formation;
(c) packer means isolating said sampling passage from wellbore pressure and ensuring communication of connate fluid into said sample probe substantially at formation pressure;
(d) a bi-directional piston pump mechanism being located within said instrument body structure and defining at least one pumping chamber having fluid communication with said fluid sampling passage, said pumping chamber further defining a testing chamber for said connate fluid, said piston pumping mechanism having a piston reciprocable within said pumping chamber, said piston defining a movable wall of said testing chamber;
(e) means for sensing piston movement and positioning within said pumping chamber and thus sensing finite volume and volume change of said testing chamber and the connate fluid contained therein;
(f) means for trapping a finite volume of connate fluid within said testing chamber;
(g) said piston being selectively movable within said pumping chamber for changing said finite volume of connate fluid contained within said testing chamber for bubble point pressure determination thereof;
(h) means for sensing the temperature of connate fluid within said testing chamber; and
(i) means for sensing the initial pressure of connate fluid within said testing chamber and for sensing pressure change of said connate fluid during volume change thereof.

Briefly, the various features of the present invention are effectively realized through the provision of a down-hole formation testing instrument that is capable of pressurizing an initial small, i.e. in the order of 70cc volume, for example, of sample fluid to a high pressure range, in the order of about $138 \times 10^3$ kPa (20,000 psi) for example. The sampling instrument is also capable of controllably depressurizing a fluid sample as well. This pressurization and depressurization capability allows for the construction of PVT relationship curves which can be used to determine the type and other desirable characteristics of reservoir fluid prior to taking a sample. PVT tests can be repeated an unlimited amount of time until a clean formation fluid sample is obtained.

The bubble point pressure and compressibility of the formation fluid is determined in order to set up the optimum sampling pressure for collecting a sample representative of the reservoir condition. At least one of the bi-directional piston pump chambers of the instrument is provided with a shutoff valve for fluid entrapment therein and is designated a bubble point pressure and fluid compressibility test chamber having a precision temperature sensor and precision fluid pressure gauge in communication therewith. The piston of the test chamber is coupled with a precision potentiometer for detection of volumetric change of the test chamber. By then selectively changing the volume of the test chamber and the connate fluid entrapped therein and by observing the pressure of the fluid as its volume is changed, the bubble point of the fluid can be observed by means of representative electronic signals. These signals are then utilized in the preparation of bubble point pressure and fluid compressibility profile plots which can then be utilized for reservoir characterization.

The compressibility and viscosity of sample fluids are evaluated from PVT curves, and utilized in the calculation of fluid flow parameters in the reservoir.

The major features of PVT services are (1) to identify the type of fluid present in the formation prior to collection of samples, (2) to accomplish determination of down-hole bubble point pressure for optimum sampling conditions, (3) provide bubble point pressure and fluid compressibility data to enable the preparation of bubble point pressure and fluid compressibility profile plots for use in reservoir characterization, (4) to achieve overpressure of the formation fluid sample to prevent phase separation on cooling and thereby provide for determination of fluid properties of the formation fluid for pressure transient analysis, and (5) to acquire down-hole PVT data to check the validity of samples at the surface, either on location or at reservoir fluid laboratories.

The apparatus of the present invention performs pressure-volume-temperature (PVT) measurement down-hole with a sampling probe of the wireline formation tester seated against the formation of interest. One of its purposes is to determine the bubble point of formation fluid/gas samples collected from the formation. Another of its purposes is conducting bubble point pressure and fluid compressibility analysis at formation depth and conditions for use in the preparation of bubble point pressure and fluid compressibility profile plots for the purpose of reservoir characterization. Before or after a sufficient amount of formation fluid is purged from the formation into either a tank or to the borehole, the instrument performs a measurement of pressure, temperature and volume of a finite sample of the formation fluid. This feature can be accomplished by the use of a pump-through feature accomplished by a draw-down system having pump-through capability. This can also be done with a separate piston which does not pump through. The basic element of the draw-down system of the instrument is a bi-directional piston pump composed of a double-acting piston and valves to control positive displacement pumping, including the direction and volume of pumping. At least one of the cylinders of the piston pump is provided with a shutoff valve for fluid entrapment and is designated as a test chamber for conducting downhole bubble point and fluid compressibility analysis at formation depth for use in reservoir characterization. Precision temperature and pressure sensors are associated with the test chamber and a precision linear potentiometer is associated with the pump piston for detection of volumetric changes of the test chamber and the connate fluid entrapped therein during testing. The sensors and potentiometer provide electronic signal output which is utilized in bubble point pressure and compressibility profile plots which are interpreted for reservoir characterization. Hydraulic pressure provided by an on-board hydraulic pump is used to provide the power for piston movement of the pump. Solenoid valves, pilot valves, and/or check valves are used to control the piston pump action and direction and to control fluid entrapment for bubble point pressure and fluid compressibility analysis.

The primary pump element is a double-acting, bi- directional, positive displacement pump incorporating a reciprocating piston which provides pumping action in both stroke directions. This differs from existing drawdown piston pump designs, where the draw-down piston only provides pumping action in one direction. This capability allows for much more rapid pumping action when compared with the conventional one- directional pumping action. Conventional draw-down units provide pumping action in one piston movement direction, and a no-action return for the other piston direction.

The pump unit can be set to pump from bottom to top, or from top to bottom. This pumping direction is accomplished in situ by controllably positioning a four-way valve. This valve is controlled by an on-board computer which is contained in the downhole instrument.

The apparatus of the present invention achieves a controlled rate draw-down. The pump displacement rate can be controlled, thus allowing a controlled rate for pressure drop, total volume, or volume/second. This control allows improved test data by adapting the test to the formation characteristics.

The apparatus of this invention is also capable of pumping fluid from the formation of interest to the borehole. This may be beneficial in removing all filtrate, i.e., mud-cake from the formation before sampling or taking other data such as PVT. Resistivity and/or capacitance can also be measured while pumping. The pump can be set to control the fluid withdrawal rate of the drawdown module. The apparatus is also capable of achieving pumping in a direction opposite to the normal direction, i.e., from top to bottom rather than from bottom to top.

Piston displacement can be acceptably measured by a variety of means that are available within the state of the art. For example, piston displacement with a position sensor displacement can be detected by measuring piston movement by means of a variable resister or an inductive coil by the time required for an acoustic pulse to echo from one face of the pump piston or by the phase shift of a light beam when reflected from one face of the pump piston. Additionally, the volumetric displacement of the piston pump can be calibrated in the down- hole environment according to the mechanical limits for movement of the pump piston in either direction. By precisely measuring piston displacement, full control of the pump can be achieved. The reverse pumping capability of the pump system allows an unlimited volume of fluid to be pumped out of the sampling probe to clean any debris such as fine sand, rocks, filtrate, etc. that may exist on the sampling probe. The reversible pump direction also allows fluid to be injected from the tool or borehole into the formation. One example may be to inject completion fluid stored in a tank, or, fluid retrieved from the wellbore, or fluid previously pumped from the formation, into the formation. After injection of the completion fluid, additional draw-downs and/or sampling may take place to determine the effect of the completion fluid on the formation of interest. So that the manner in which the above recited features, advantages and objects of the present invention are attained and can be understood in detail, a more particular description of the invention, briefly summarized above, may be had by reference to the embodiments thereof which are illustrated in the appended drawings.

It is to be noted, however, that the appended drawings illustrate only typical embodiments of this invention and are therefore not to be considered limiting of its scope, for the invention may admit to other equally effective embodiments.

Fig. 1 is a pictorial view, partly in cross-section, of a formation test instrument constructed in accordance with the present invention and disposed in a borehole in sampling position with respect to a formation of interest and further showing a surface processing and control system therefor by way of block diagram schematics.

Fig. 2 is a sectional view of a portion of the downhole multi-testing tool of Fig. 1, illustrating the bi-directional piston draw-down assembly thereof in position within the pump compartment of the instrument body.

Fig. 3 is a sectional view of the bi-directional piston pump mechanism of Fig. 2.

Fig. 4A is a sectional view of a pilot operated four- way formation fluid check valve assembly for selecting and reversing the pumping direction of the bi-directional piston pump mechanism of Fig. 3 and being shown in the normal position thereof.

Fig. 4B is a sectional view of the pilot operated four- way formation fluid check valve assembly of Fig. 4A, with the valve mechanism being shown in the pilot operated position thereof.

Fig. 5 is a hydraulic schematic illustration of the bi- directional piston pump mechanism of Fig. 3 together with hydraulic control circuitry for changing the direction of pump through while the sampling instrument is located downhole.

Fig. 6 is a partial hydraulic schematic illustration representing the addition of a pilot operated valve for confining a sample for PVT testing and illustrating solenoid control valving for positioning this valve.

Fig. 7 is a hydraulic schematic illustration of fluid circuitry for operation and control of the double-acting, bi- directional formation draw-down and pump through system of this invention.

Fig. 8 is a graphical representation illustrating the use of a pressure vs volume plot to determine the bubble point and compressibility of a fluid.

Fig. 9 is a graphical representation illustrating the use of bubble point pressure vs formation depth to identify different subsurface production zones.

Fig. 10 is a graphical representation illustrating how adding compressibility plotted vs depth can identify different subsurface fluid production zones which have the same fluid pressure.

Fig. 11 is a mechanical and electrical schematic illustration of a portion of the bi-directional piston pump mechanism of the present invention which constitutes a test chamber and system for conducting bubble point pressure and fluid compressibility analysis at formation depth.

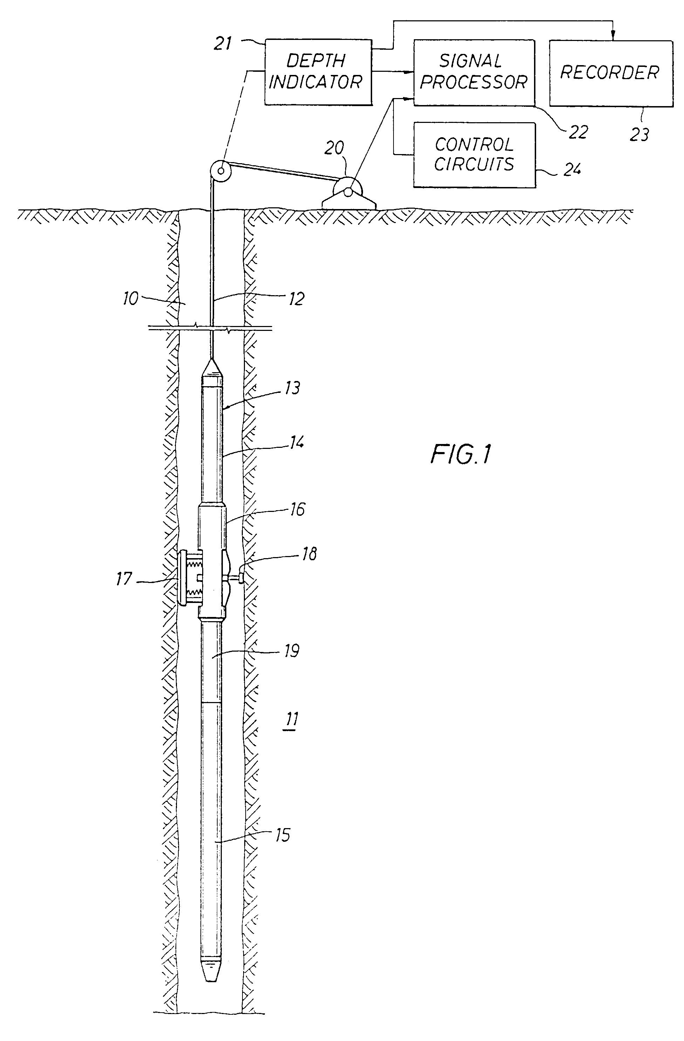

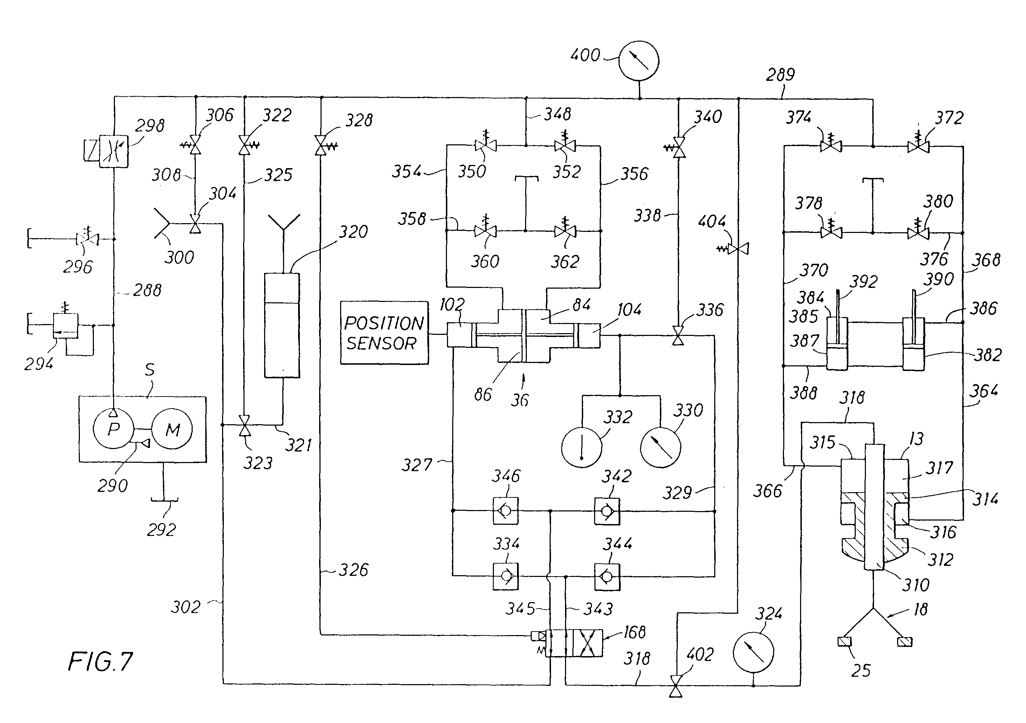

Referring now to the drawings in more detail, particularly to Fig. 1, there is illustrated schematically a section of a borehole 10 penetrating a portion of the earth formations 11, shown in vertical section. Disposed within the borehole 10 by means of a cable or wireline 12 is a sampling and measuring instrument 13. The sampling and measuring instrument is comprised of a hydraulic power system 14, a fluid sample storage section 15 and a sampling mechanism section 16. Sampling mechanism section 16 includes selectively extensible well engaging pad member 17, a selectively extensible fluid admitting sampling probe member 18 and bi-directional pumping member 19. The pumping member 19 could also be located above the sampling probe member 18 if desired. The sampling probe will include one or more packers, as shown schematically at 25 in Fig. 7, for engaging the well bore and isolating the fluid inlet passage thereof from the wellbore pressure which is typically significantly higher than formation pressure. Any other suitable packer systems may be employed for isolating the sampling probe from wellbore pressure to thus ensure that formation pressure or a pressure varying only slightly therefrom is introduced through the sampling probe to the various fluid circuits of the multi tester tool.

In operation, sampling and measuring instrument 13 is positioned within borehole 10 by winding or unwinding cable 12 from hoist 20, around which cable 12 is spooled. Depth information from depth indicator 21 is coupled to signal processor 22 and recorder 23 when instrument 13 is disposed adjacent an earth formation of interest. Electrical control signals from control circuits 24 are transmitted through electrical conductors contained within cable 12 to instrument 13.

These electrical control signals activate an operational hydraulic pump within the hydraulic power system 14 shown schematically in Fig. 7, which provides hydraulic power for instrument operation and which provides hydraulic power causing the well engaging pad member 17 and the fluid admitting member 18 to move laterally from instrument 13 into engagement with the earth formation 11 and the bi-directional pumping member 19. Fluid admitting member or sampling probe 18 can then be placed in wellbore pressure isolated fluid communication with the earth formation 11 by means of electrical controlled signals from control circuits 24 selectively activating solenoid valves within instrument 13 for the taking of a sample of any producible connate fluids contained in the earth formation of intent.

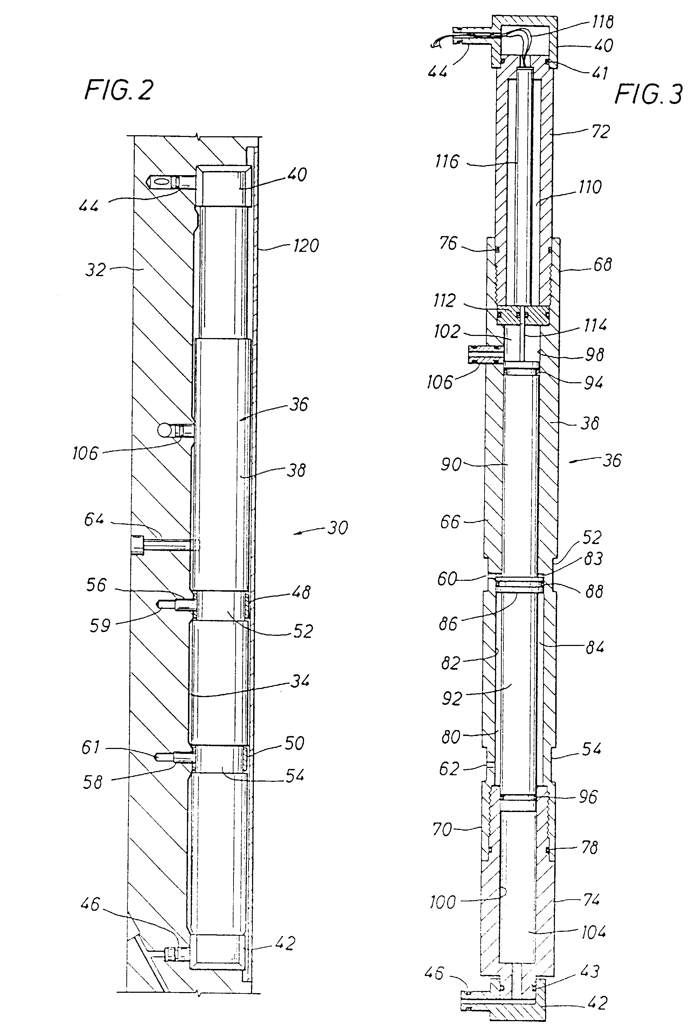

Referring now to the partial sectional view of Fig. 2, there is illustrated a double ended, bi-directional pumping section of the sampling instrument, shown generally at 30 which will typically comprise a component part 19 of the multi-tester instrument. The pumping section 30 incorporates a double-acting bi-directional fluid control system having the capability of injecting a fluid medium such as completion fluid for example, into a formation of interest and for withdrawing connate fluid from the formation. This feature permits downhole testing of the effect of a selected completion fluid on the formation intended to be completed for production. This apparatus is also capable of pumping injection fluid either from the wellbore or from a reservoir contained within the instrument and injecting the fluid into the formation. Additionally, the double-acting, bi-directional fluid control system has the capability of withdrawing connate fluid from the formation of interest and selectively discharging the fluid into the wellbore, such as for purging the system of debris such as fine sand, rocks, filtrate, and other foreign matter or selectively conducting the recovered connate fluid through the sampling system of the instrument. The bi-directional fluid control system is also constructed to define a test system for conducting bubble point pressure and fluid compressibility testing at formation depth. These and other features of the invention will become evident as the preferred embodiment of this invention is described in detail hereinbelow.

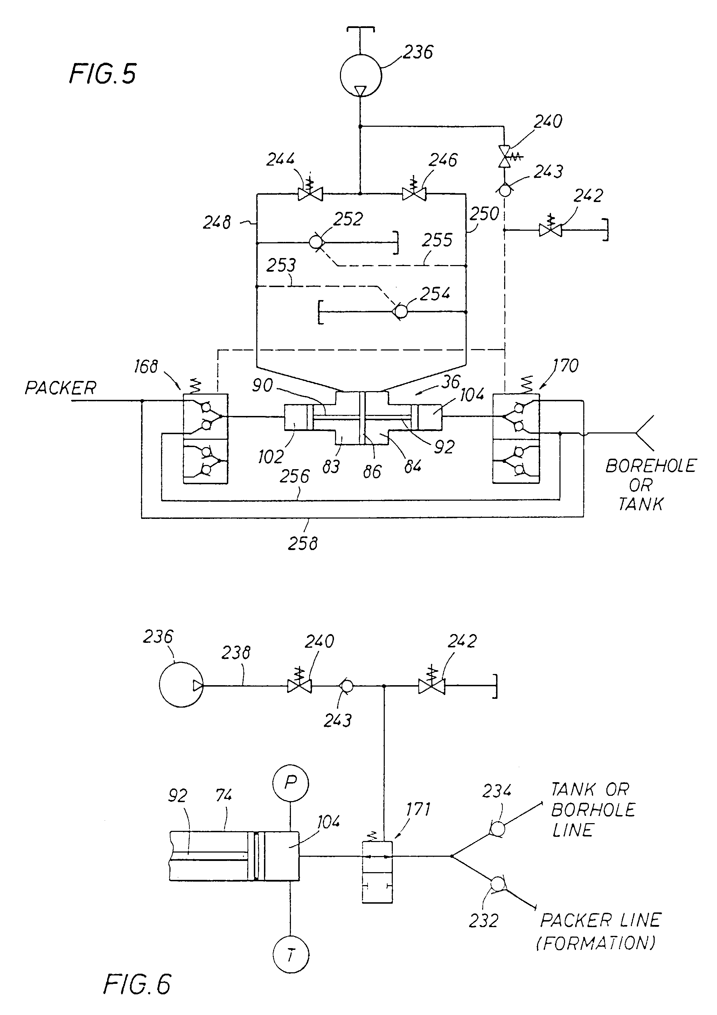

The pumping section 30 defines an elongate body structure 32 which is intended for use alone or in mechanically coupled relation with other sampling instrument components. This structure would contain appropriate cavities and passageways to accomplish the pumping circuitry illustrated in Figs. 5, 6 and 7.

As shown in Fig. 2 the body structure 32 defines a pump cavity 34 within which is located a bi-directional piston pump shown generally at 36 and being shown in greater detail in Fig. 3. The piston pump 36 incorporates an elongate pump body 38 having upper and lower end connection members 40 and 42 which are sealed to the terminal body sections by annular seals 41 and 43. The end connection members are restrained against displacement from their sealed assembly with the terminal body sections by the body structure 32 or by other suitable means and are coupled by tubular projections 44 and 46 thereof which are received in sealed relation with body receptacles of the body structure 32. The pump 36 is also retained within the cavity or compartment 34 by means of retainer clamps 48 and 50 which are received about reduced diameter portions 52 and 54 of the pump body 38 and are secured to the body structure 32. The retainer clamps each function as connecting rings and serve to retain quick disconnect couplings 56 and 58 in sealed assembly with the body 32 and the pump body 38 so as to establish fluid communication between pump body operating fluid inlet and outlet ports 60 and 62 and respective fluid passages of the body 32. The intermediate portion of the pump body 56 is also retained in secured assembly with the body structure 32 by means of a retainer screw or bolt 64 which extends through a bolt opening in the instrument body 32 and is threadedly received by the pump body 38.

The pump body structure 38 incorporates an intermediate body section 66 as best seen in Fig. 3 having internally threaded extremities 68 and 70 respectively to which are threadedly connected terminal body sections 72 and 74. The terminal body sections are sealed with respect to the intermediate body section 66 by means of annular seals 76 and 78 respectively.

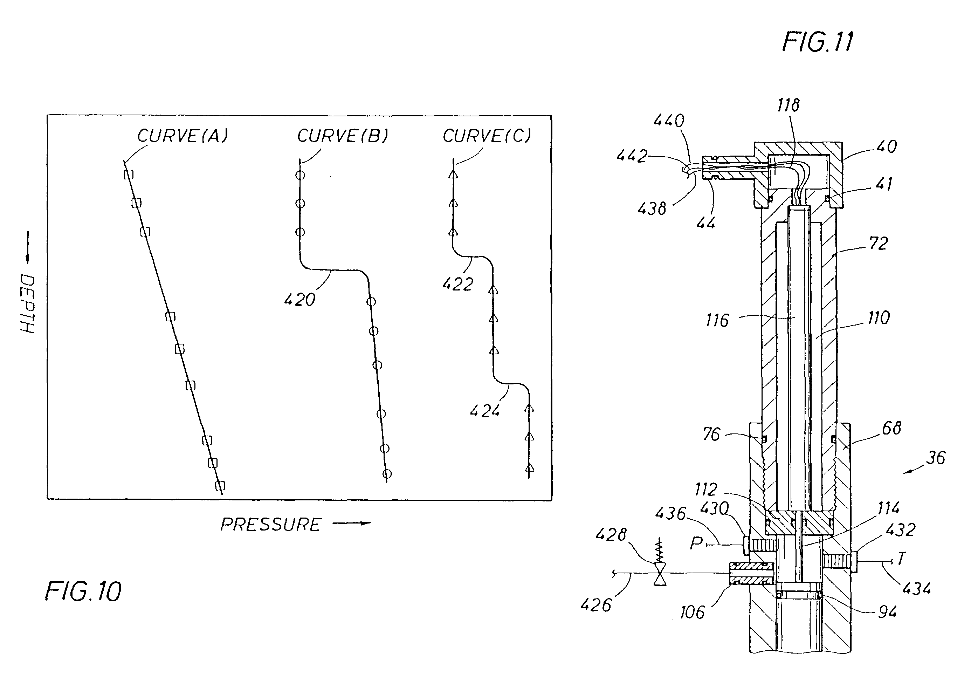

The intermediate body section 66 and the terminal body section 74 cooperatively form an elongate internal pump chamber 80 having an enlarged intermediate piston section 82 forming a piston pumping chamber. A piston 86 partitions the pumping chamber 80 into variable volume pumping chambers 83 and 84 on respective sides of the piston. The piston is sealed with respect to the cylindrical pumping chamber surface 82 by means of an annular piston seal 88 and is reciprocated within the pumping chamber by hydraulic fluid selectively injected through pump chamber inlet ports 60 and 62 into respective pumping chambers 83 or 84. From opposite sides of the piston 86 extend piston stems 90 and 92, each supporting respective high pressure seal assemblies 94 and 96 having sealing engagement with respective internal cylindrical surfaces 98 and 100 which respectively define pumping chambers 102 and 104. These pumping chambers are in communication respectively with tubular connector elements 46 and 106 which function to provide sealed fluid interchange between the respective pumping chambers of the piston pump and formation fluid flow passages which are defined by the instrument body structure 32. One of the pumping chambers may be designated as a valve controlled test chamber, as illustrated in greater detail in Fig. 11, for entrapping a known volume of connate fluid and conducting bubble point pressure and compressibility testing of the fluid so that the test results may be employed in bubble point pressure and compressibility profile plots for reservoir characterization. Tubular connector element 106 is sealed with respect to the pump body and the instrument body by means of annular seals. Thus, as hydraulic fluid functions to impart reciprocation to the piston 86 and its piston stems 90 and 92, the piston stems accomplish suction and pumping of formation fluid depending upon the direction of piston movement. As one pumping piston is moving in its power stroke for forcible ejection of formation fluid from its pumping chamber, the opposite piston will be moving in its suction stroke, drawing formation fluid into its pumping chamber for subsequent pumping displacement.

Terminal housing section 72 defines an internal position sensor chamber 110 which is sealed with respect to the pumping chamber 102 by means of a partition seal assembly 112. A position indicator stem 114 projects from the end portion of piston stem 90 and extends in sealed relation through the partition seal assembly 112 and into an internal receptacle defined within a position sensing potentiometer or other suitable piston position sensor 116. The output signals of the precision potentiometer are effectively used to determine known test chamber volume and volumetric change for the purpose of bubble point pressure and fluid compressibility testing. Electronic signals representing the position of the position indicator stem 114 within the potentiometer 116 are transmitted via electrical conductors 118 which extend through the tubular connector projection 44 to appropriate electrical circuitry within the instrument body 32. Thus, as the piston 86 is reciprocated within its chamber 84 this piston movement is sensed and transmitted electronically where it may be utilized for pumping control for pump calibration and for precision volumetric measurement, such as for determination of known volume and volumetric change of the test chamber.

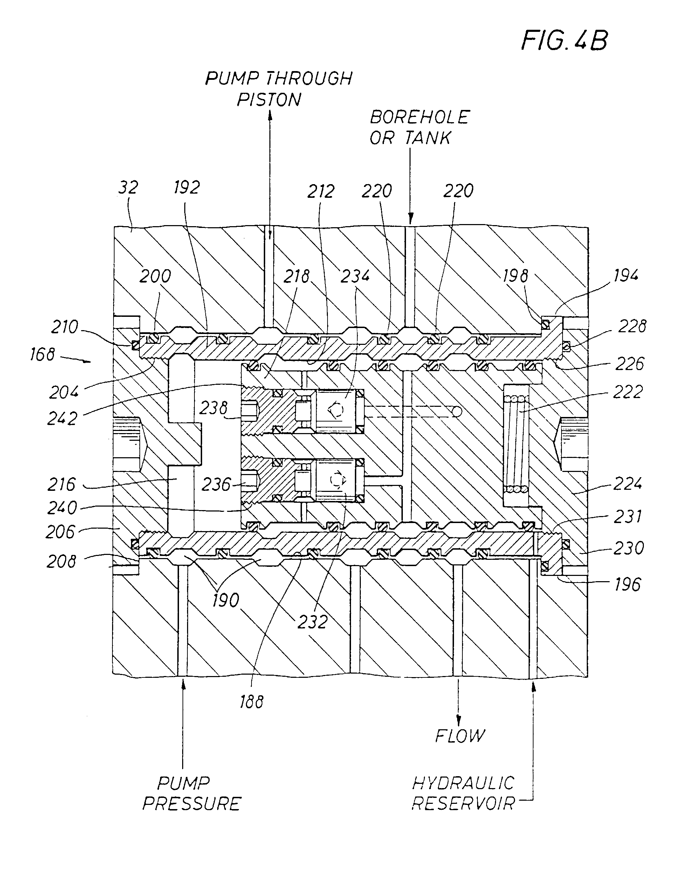

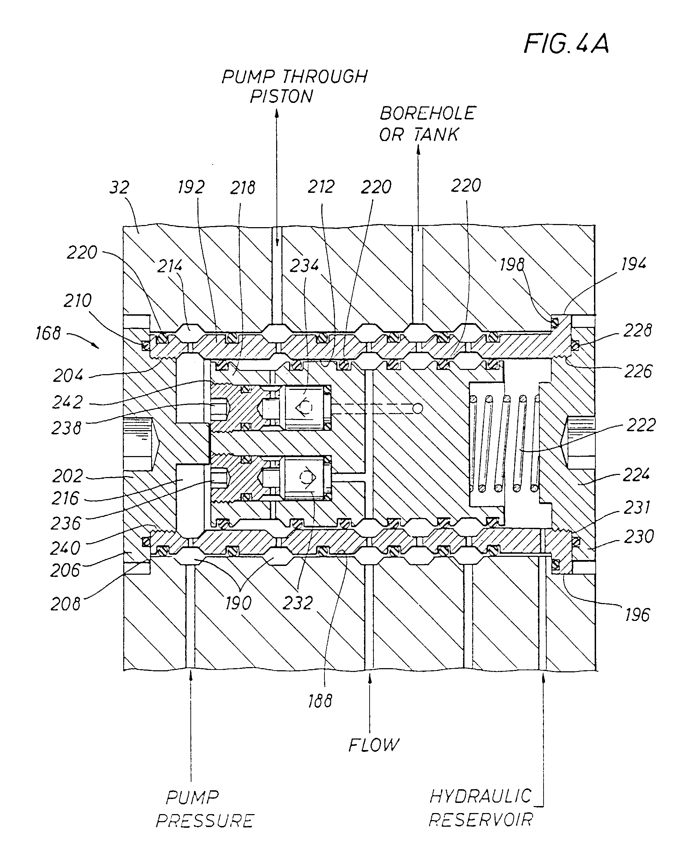

The piston pumping capability achieved by the piston stems or plungers 90 and 92 is relatively small but this piston pumping activity may be achieved at significantly high pressure, i.e., in the order of $138 \times 10^3$ kPa (20,000 Psi), by controlling the relative pressure responsive dimensions of the piston 86 and the pump cylinders 98 and 100. In the event lower pressure, higher volume pumping is considered appropriate, a double-acting pump mechanism may be provided having larger diameter pump bores and piston plungers as compared with that shown in Fig. 3. In fact, since the double-acting, bi-directional pump mechanism 36 is preferably releasably secured within its cavity or receptacle, changing the pumping capacity of the modular bi-directional pumping section 30 may be simply achieved even under field conditions. After the protective cover 120 has been removed, the piston pump assembly 36 will be exposed and may be simply and quickly removed through the use of readily available tools and replaced with a pump of different pumping capacity. Thus, if a different capacity pump is desired, or if the piston pump is in need of repair or replacement for any reason, this can be simply and efficiently accomplished even under field conditions in only a few minutes time, through the use of ordinarily available tools. This feature also permits a downhole multitester instrument without bubble point pressure testing capability to be readily converted, simply by changing out the bi-directional pump mechanism. Sealed fluid interconnection between the piston pump 36 and respective fluid passages of the body structure 32 may be of the plug-in type so as to simplify the installation and removal procedure for the pump. Each of the end fittings 40 and 42 are provided with connection projections of the plug-in type such as shown at 44 and 46 which are each provided with annular sealing elements for establishing seals within respective bores of the pump body. Likewise, the intermediate section of the piston pump is provided with a tubular connection fitting 106 having annular seals in seal grooves thereof for establishment of sealed interconnection with both the intermediate body section of the pump and the instrument body 32. In order to enable reversal of the pumping direction of the bi-directional pump mechanism 36 a pair of pilot operated control valve assemblies 168 and 170 are provided which are illustrated schematically in Fig. 5 and shown in the sectional view of Figs. 4A and 4B. These control valves are generally referred to herein as "dirty fluid check valves" since they are employed to control the directional pumping of formation sample fluid and injection fluid through the various fluid passages of the instrument. Although these control/check valves 232 and 233 are of somewhat differing construction, they are mounted in a piston assembly 218 which is shifted hydraulically under selective control from the surface such as by control circuits 24 or are shifted between operating positions under programmed control by control circuitry which is either located within the surface based circuits or within the instrument itself. From a standpoint of basic construction, the dirty fluid check valve mount assembly is generally in the form shown and described in connection with Figs. 4A and 4B. The dirty fluid check valve shown generally at 168 and 170 in Fig. 5 is a hydraulically operated four-way check valve which has the function of changing the pump-through direction of the sampling instrument. A two way normally open hydraulically operated valve 170 shown in Fig. 6 is used to enable in situ PVT testing by the instrument.

With reference now to Fig. 4A, a representative example of the dirty fluid valve 168 is illustrated, this being a pilot operated four-way valve. The instrument body 32 defines a plurality of fluid flow passages which are labeled as indicated, which passages are disposed in communication with a transverse valve bore 188. At the juncture of the respective passages with the valve bore, the valve bore is enlarged to define annular fluid conducting grooves such as shown at 190. A valve seat sleeve 192 is positioned within the bore 188 and defines a shoulder flange 194 which is seated against a circular internal stop flange 196 defined by an enlargement at one end of the bore 188. The valve sleeve is sealed with respect to the instrument body by means of an annular sealing element 198 which engages the stop shoulder 196. At its opposite end the valve sleeve 192 is sealed with respect to the instrument body 32 by means of an annular sealing element 200 which is seated against a portion of the cylindrical internal sealing surface defined by the valve bore 188. A valve retainer cover 202 is provided with an externally threaded section 204 which is received by an internally threaded internal portion of the valve sleeve 192 and defines an annular retainer flange 206 which engages an internal annular shoulder 208 to lock the valve sleeve in sealed, static position within the valve bore 188. The valve cover is sealed with respect to the valve sleeve 192 by means of a circular sealing element 210 which establishes sealing engagement with one end wall of the valve sleeve. The valve sleeve is of tubular configuration and defines an internal, cylindrical segmented sealing surface 212 which is interrupted by a plurality of internal fluid transfer grooves 214 and which establishes an internal valve chamber 216 within which is positioned a generally cylindrical valve spool element 218. The valve spool has sealed relation with respect to the internal cylindrical sealing surface segments 212 by means of a plurality of circular sealing elements 220.

The valve element of Figs. 4A and 4B, 218 is shown to be urged in one direction by means of a compression spring 222 having one end thereof in force transmitting engagement with the valve element 218 and with the opposite end thereof in restrained engagement with a closure and retainer element 224 which establishes threaded connection with the valve sleeve at 226. An annular sealing element 228, carried by a circular seal groove in a radial sealing flange portion 230 of the retainer and closure element 224, establishes sealing engagement with an annular planar end surface 231 of the valve sleeve. It should be born in mind that the valve spool may be springless so that it does not receive a mechanically induced urging force. In the alternative, a similar valve may be provided having a valve spool that is hydraulically energized for movement in either direction and which is releasably retained at each of its positions by means of retainer detents. In the form of the invention shown in Fig. 4A, the valve spool element 218 is shown to define internal check valve chambers within which are shown a pair of check valve elements 232 and 234 by way of schematic illustration. The check valve elements 232 and 234 are retained within the valve spool by means of a pair of check valve retainers 236 and 238 which are threadedly received by outer, internally threaded portions 240 and 242 of the check valve receptacles. The closures 236 and 238 are sealed with respect to the valve spool element 218 by means of external annular sealing elements provided in seal grooves thereof.

As further shown in Fig. 4A, the valve spool element 218 is shown in the position for achieving flow from the packer to the borehole or to a sampling tank or vessel disposed internally of the instrument. As the piston pump mechanism is operated, therefore, movement of the bi-directional piston in one direction achieves suction induced flow of production fluid from the packer, tank or other source into the pump chamber. Since the packer is usually sealably disposed against a formation, this fluid is usually filtrate or formation fluid. If the packer is not seated, wellbore fluid would be pumped. By placing a cup shaped elastomeric seal around the tool between the packer and the wellbore exhaust port, drilling mud could be pumped by the bi-directional piston pump to urge the tool uphole or down-hole. This bi-directional pump is the first practical means to develop sufficient pressure (0.7 x 10³ kPa (100 Psi) approximates tool weight in 171.45 mn (6-3/4)hole) to lift or otherwise shift a tool that has become differentially stuck. The output of the piston pump could also go to a pair of inflatable packers prior to being used to pump fluid into or from the "packed off zone". Adding large volume tanks could provide a small scale drill stem test. U.S. Patent No. 4,535,843 broadly describes the pumping of fluid but does not practically address how to construct or control such a pump. When the direction of the piston is reversed, this recovered formation fluid is then pumped either to the wellbore such as for flushing away fine sand, rocks, mud-cake or other debris that is present at the juncture of the sampling probe with the borehole wall at formation level. After all of the debris has been flushed into the wellbore, pumped flow is altered to permit pumping of formation fluid from the sampling probe into one or more sampling vessels for on-board storage, for subsequent down-hole disposal or for ultimate transfer to the surface for laboratory testing.

The valving apparatus also has the capability of achieving pumping of liquid constituents, such as completion fluid, oil-water mixtures, either from a fluid reservoir in the instrument, or drilling mud from the borehole directly into the formation. This feature effectively enables the formation to be tested with a completion fluid so that the effect of the completion fluid may be determined prior to actual completion of the well or relative permeability to known viscosity fluid determined. Reversal of the direction of fluid pumping is achieved by applying hydraulic pressure from an operating pressure source located in the instrument to the pump pressure inlet passage shown at the upper left hand portion of Fig. 4B. Pressurized hydraulic fluid thus forces the valve spool element 218 downwardly, compressing the spring 222. Fluid present within the valve chamber 216 below the sleeve valve will be conducted through the sleeve valve to the hydraulic reservoir passage located at the lower left hand portion of Fig. 4B for return to the sump of the hydraulic fluid pump. In the position shown in Fig. 4A, operation of the bi-directional piston pump mechanism will induce fluid flow through the packer/sample passages of the valve mechanism and through the check valve 232 as the pump piston moves in one direction. As the direction of the pump piston is reversed, flow then is achieved through the opposite check valve 234, thereby forcing the collected formation sample through the borehole or tank passage shown at the lower right hand portion of Fig. 4A. In the valve position shown in Fig. 4B the pumped fluid will flow through the borehole/tank passages of the valve mechanism and through the check valves 232 and 234 to the packer/sample passages of the instrument body as shown at the lower left hand portion of Fig. 4B. Flow of the collected formation sample to the borehole or sample tank is controlled by appropriate electronically selected, electrically or hydraulically energized valving.

Referring now to Fig. 6, there is shown a simplified schematic illustration of a portion of the downhole instrument to perform pressure-volume-temperature (PVT) measurement down-hole with the wireline formation tester while seated against the formation. In cases where differential sticking is a problem, the sample could be taken into a tank after which the tool can be closed and moved slowly up or down the borehole while PVT analysis is conducted on the fluid in the sampling tank. One of its purposes is to determine the bubble point pressure and fluid compressibility of fluid/gas samples collected from the formation of interest so that the formation or formations may be characterized through use of this information. Before or after a sufficient amount of formation fluid is purged from the formation into either a tank or to the borehole, the multitester instrument can be controlled to perform a measurement of pressure, temperature and volume of a finite sample of formation fluid. This is accomplished by the use of the double-acting, bi-directional pump mechanism which includes a pump-through capability. The simplified schematic illustration of Fig. 6 discloses a hydraulic operating pressure supply pump 236 which discharges pressurized hydraulic fluid through a pilot pressure supply conduit 238 under the control of a pair of solenoid valves 240 and 242 and a check valve 243. These normally closed solenoid valves are selectively operated to direct the flow of hydraulic fluid from the hydraulic pump 236 to a normally open, two-way dirty fluid valve, shown generally at 171. A portion of the bi-directional pump mechanism of Fig. 3 is illustrated, showing one of the pistons 92 being reciprocable within the piston chamber 104. Pressure and temperature sensors "P" and "T" are in communication with the piston chamber 104 to thereby permit in situ inspection of the pressure and temperature of the formation fluid which is present within the piston chamber. Since the dirty fluid valve assembly 171 is a normally open, two-way valve, in its open position as shown in Fig. 6, the pumped fluid from the piston chamber 104 is delivered through the valve assembly 170 to the check valves 232 and 234. When the piston 92 shown in Fig. 6 is moving to the left, it develops suction in the pumping chamber 104 which acts through the normally open valve 171, thereby inducing flow of formation fluid through the sampling probe and packer line from the formation of interest and across the check valve 232. When the piston 92 of the bi-directional pump mechanism is moving in the opposite direction, its discharge flow is achieved through the dirty fluid valve 171 and through the check valve 234 to the tank or borehole line. The flow of fluid to the sample collection tank or to the borehole is selected by using a solenoid control valve to shift a two-way dirty fluid valve that is located in a different section of the instrument.

As shown in the simple schematic illustration of Fig. 5, the bi-directional pump mechanism is illustrated generally at 36 with its pumping chambers 102 and 104 coupled in fluid communication with the dirty fluid check valve assemblies shown generally at 168 and 170. The bi-directional pump mechanism is capable of pumping from either of its pumping chambers 102 and 104 to the packer line or to the borehole- storage tank line, depending upon the position of the dirty fluid check valve mount assembly, as controlled by the positions of the respective solenoid valves 240 and 242 of the pilot pressure supply line 238. For reciprocating operation of the bi-directional piston pump mechanism, directional control valves 244 and 246 are selectively opened by an electrical control circuit, thereby directing pump pressure selectively to the pump pressure supply lines 248 and 250. Check valves 252 and 254 are provided in return line circuits to conduct hydraulic fluid from the respective piston chambers 83 and 84 to the hydraulic storage reservoir of the hydraulic supply pump 236 and are selectively propped open by pressure via broken pilot pressure lines 253 and 255. A pair of directional flow lines 256 and 258 are coupled respectively to the packer line and to the borehole or supply tank line and function to direct pumped formation fluid or completion fluid from the respective pumping chambers 102 and 104 in a direction selected by the position of the dirty fluid check valve mount assemblies 168 and 170. By simply reversing the direction of pumping, the bi-directional, double-ended pump 54 has the capability of pumping fluid either into the formation or from the formation and pumping collected formation fluid either into a sample collection vessel or into the wellbore. These features provide significant advantage from the standpoint of downhole testing flexibility.

An alternative method for reversing flow is illustrated in Fig. 7. In this case a four-way valve switches inlet/outlet lines to rigidly mounted dirty fluid check valves. The hydraulic circuitry of the wireline downhole testing instrument is illustrated schematically Fig. 7 and shows a hydraulic fluid supply line 288 being the discharge line of a hydraulic fluid pump "P" which is driven by an electric motor "M". The electric motor is powered and controlled through appropriate electrical circuitry from the surface based equipment shown in Fig. 1. The pump "P" derives its source of hydraulic fluid from a sump "S" via a suction line 290. The pump and motor are preferably contained within the hydraulic fluid reservoir which is the sump for the purpose of cooling, but such is not intended to limit the scope of this invention. The symbol "292" where it occurs in the hydraulic circuitry, represents the return of hydraulic fluid to the sump "S".

Pressure within the supply line 288 is limited by a pressure relief valve 294 which relieves excessive pressure to the sump. Pressure in the hydraulic supply line 288 is selectively vented to the sump upon operation of a normally closed solenoid valve 296. The line pressure of supply line 288 is selected for desired pressure level for operation of the various hydraulic fluid circuits of the downhole sampling instrument by means of an electrically operated variable flow resistor 298.

For PVT testing of the formation of interest it is appropriate to establish communication of the sampling probe or admitting member 18 with the subsurface formation of interest which is traversed by the wellbore. Symbol "300" is representative of the wellbore at formation level. Admission of fluid to and from the wellbore is accomplished by means of a sampling circuit 302 having a pilot operated isolation valve 304 to which hydraulic pressure is supplied via a solenoid valve 306 in branch hydraulic fluid supply line 308.

The fluid admitting member shown generally at 18 includes a movable sampling probe 310 which is movable laterally from the instrument 13 in conjunction with a hydraulically energized probe actuating piston 312 having its piston portion 314 received within a hydraulic cylinder 316 that is provided within the instrument body and partitioning the cylinder into hydraulic chambers 316 and 317. The sampling probe 310 is hydraulically energized independently of the piston 312. A formation fluid line 318 in communication with the fluid passage of the sampling probe 310 is coupled with a pilot operated four-way dirty fluid check valve assembly 168. This valve, when positioned as shown in Fig. 7, establishes connection of line 318 with suction, discharge lines 327 and 329 via check valves 334 and 344 depending upon the direction of movement of the pump piston 86. The sample line 302 is connected to the suction discharge lines of the pump 36 via line 345 and check valves 342 and 346 through the dirty fluid check valve assembly discussed hereinbelow and is also coupled with at least one sample collection tank 320 under the control of a pilot operated valve 322 and an isolation valve 323. The pressure within the sample line 318 is detected by a pressure sensor 324 which may be an absolute pressure gauge as schematically illustrated or which may take any other suitable form.

It is desirable that the downhole testing and sample collection instrument have the capability of varying the pressure of the formation fluid from actual formation pressure level for the purpose of conducting certain downhole, in situ testing, such as bubble point pressure and fluid compressibility testing, and also for the purpose of conducting certain laboratory testing of the formation fluid collected within the sample collection tank 320. For example, by withdrawing fluid from a formation at a pressure above the bubble point pressure fluid samples which have not undergone phase separation can be recovered and delivered to a laboratory for testing by sufficiently elevating the pressure of the formation fluid above its bubble point pressure to allow cooling to surface temperature without dropping the pressure in the tank below be bubble point pressure. It is desirable, therefore, that the instrument have the capability of elevating the pressure of the collected formation fluid to a differential pressure range of about 138 x $10^3$ kPa (20,000 Psi) for this particular purpose. To accomplish pressure variation of the formation fluid sample, also referred to herein as "dirty fluid", the pilot operated four-way valve shown generally at 168 is coupled in selected, pilot controlled communication with the sample lines 302 and 318. This valve receives its pilot pressure energization from the supply line 289 via pilot pressure supply line 326 under the control of a solenoid valve 328.

The double-acting, bi-directional piston pump mechanism illustrated generally at 36 and described above in connection with Fig. 3 is connected with its respective suction/discharge chambers 102-104 in connection with dirty fluid flow lines 327 and 329 respectively. The pumping pressure of the pump mechanism is sensed by an absolute pressure gauge 324. For PVT analyses the pressure and temperature of the formation fluid is sensed by a pressure gauge 330 and a temperature sensor 332.

With the dirty fluid check valve 168 at its normally open position as shown in Fig. 7, the suction stroke of pumping chamber 102 will induce the flow of formation fluid through the sampling probe 310, sample line 318, valve 168, line 343, check valve 334 and flow line 327 to the pumping chamber 102. At the same time the double-acting piston pump 36 discharges formation fluid from the pumping chamber 104 and through the flow line 329 when the pilot operated normally closed control valve 336 is open by pressure supplied through conduit 338 upon opening of the normally closed solenoid valve 340. The formation fluid, thus pressurized by the piston pumping system, flows through check valve 342 and connector line 345 to the sample line 302. With the pilot operated valve 323 open by energization of the normally closed solenoid valve 322 via pressure supply line 325, the pressurized formation fluid is caused to enter the sample tank 320 via sample tank line 321. When the direction of the piston pump 36 is reversed, pumping chamber 104 becomes the suction chamber and pumping chamber 102 becomes the pressure chamber. In this case, the flow of formation fluid from the sample line 318 occurs through the valve 168 and check valve 344 and through line 329 and open valve 336 into the chamber 104. At the same time, the piston of the piston pump 36 develops pressure in pumping chamber 102 thereby discharging pressurized formation fluid through line 327 and check valve 346 to the dirty fluid check valve 168 through line 345. This pressurized formation fluid is conducted to the sample tank 320 under circumstances when the pilot operated valve 323 is open by introduction of pilot pressure through line 325 when solenoid valve 322 is energized to its open position. At this time pilot operated valve 304 will be closed, thus isolating the pressurized formation fluid from the formation. For discharge of the fluid being pumped through sample line 302 into the wellbore valve 304 will be opened by pilot pressure supplied through open solenoid valve 306 while tank supply solenoid valve 323 will be closed.

As the piston pump 36 is operated, the position of its piston 86 is precisely detected at all times by the position sensor shown schematically in Fig. 7 and shown at 114-116 in Fig. 3. This feature permits precision measuring of piston displacement versus time of displacement and also permits precision measuring of finite test chamber volume and volumetric change for the purpose of determining bubble point pressure and fluid compressibility of the connate fluid. The precision pressure and temperature gauges 330 and 332 also provide precise pressure and temperature data to provide the instrument with precision volumetric measurement of fluid being extracted from the formation. Additionally, the double-acting, bi-directional piston pump may be effectively calibrated in the downhole environment to achieve pumping at a rate that is determined by formation production capability. To accomplish this feature, the actuating fluid system of the piston pump 36 receives hydraulic pumping pressure via branch supply line 348 upon selective controlled opening and closing of the pump actuating solenoid valves 350 and 352 thus selectively pressurizing pump operating pressure supply lines 354 and 356. Obviously when one supply line 354 or 356 is pressurized to thus pressurize one of the piston pumping chambers 83 or 84, the opposite piston pumping chamber must be vented to permit fluid displacement. This feature is accomplished through the provision of a vent line 358 which is coupled to the sump and which is controlled by selectively opened solenoid valves 360 and 362. Thus, the solenoid valves 350, 352, 360 and 362 are cycled electronically so as to achieve selective operation of the piston pump 36. Further, these valves are selectively controllable electronically so as to achieve precision piston reciprocation to thereby achieve precision volumetric fluid measurement capability. Since the precise position of the piston 86 is known at all times, data reflecting the rate of piston movement is also indicative of the rate of fluid pumping into the formation or the rate of fluid recovery from the formation. Precision piston detection also permits downhole calibration of the pump to be accomplished by electronically adjusting cycling of controls and/or adjusting the pressure of the hydraulic fluid supply, such as by varying the pressure control of the electronically controlled fluid flow resistor 298.

Upon selective opening of the solenoid valve 328 for pressurization of the pilot pressure supply line 326 the four-way dirty fluid check valve 168 will be shifted to its reverse flow position so that pumped fluid under pressure from either of the pumping chambers 102 or 104 will be delivered through check valves 342 and 346 respectively to the connector line 345 where it is conducted through the shifted dirty fluid check valve 168 and caused to flow through sample line 318 to the sampling probe 310 and thence into the formation. The fluid being injected into the formation may be recovered from the sample tank 320 via open valve 323 and sample line 302, thus precluding any necessity to retrieve the sampling instrument for the purpose of sample collection disposal. Further, with the valve 168 in its normal position as shown in Fig. 7, and with the valve 323 closed and valve 304 open, samples of formation fluid being pumped may be delivered into the wellbore to thus provide a virtually unlimited flushing capacity to remove filtrate from formation. By reversing the dirty fluid check valve 168 and with valve 304 open, collected formation fluid, perhaps including certain testing fluids such as completion fluid, may be recovered from the wellbore and reinjected by the bi-directional piston pumping mechanism through the sampling probe 310 into the formation. For this reason, disposal of formation fluid or sampling fluid or a combination of the two, i.e., filtrate, may be disposed of by pumping it into the formation for dispersal into the fluid medium contained within the formation. Further, the instrument is capable of pumping virtually unlimited quantities of testing fluid, such as completion fluid from the wellbore into the formation or pumping testing fluid from on-board storage tanks into the formation and then recovering treated formation fluid for in situ testing and/or for laboratory testing.

For operation of the sampling probe the piston energized plunger 312 is operated by injection of pressurized fluid into respective piston chambers 316 or 317 via supply conduits 364 and 366 respectively. For this purpose, hydraulic pressure is supplied via supply line 289 which contains pressure regulated hydraulic oil. Valve 298 regulates the pressure in supply line 289 from surface setting via control circuit 24 and electronic feedback control to control the pressure detected by supply line pressure gauge 400 to branch supply lines 368 and 370 via solenoid energized control valves 372 and 374. Obviously, when one of the piston chambers is being supplied with pressurized hydraulic fluid, the opposite piston chamber must be vented. For this purpose, a hydraulic vent circuit 376 is coupled across conduits 368 and 370 and is vented to the hydraulic sump upon selective opening of solenoid valves 378 and 380. A pair of backup pistons 382 and 384 are coupled to piston supply lines 386 and 388 so as to simultaneously supply selected backup piston chambers 385 and 387 with hydraulic pressure. The piston stems 390 and 392 of the backup pistons impart force to operating plungers for backup plate 17 of Fig. 1.

With valving set as in Fig. 4A, a small volume change (in the range of from 5 cc to 20 cc and preferably about 10cc) can be made. This change would be called a "draw down" test. In this test, which is commonly run with current testers, the pressure in the instrument as measured by gauge 324 shown at the bottom right hand portion of Fig. 7 first decreases below formation pressure and then increases or "builds up" to formation pressure as flow from the formation repressurizes volume of fluid between the formation and the piston chamber 104 or 102. The larger this volume, the longer is the time required for "build up" to formation pressure. It is desirable to reduce the total time required for pressure testing; therefore, the volume between the formation and the displacement needs to be as small as possible. Practical considerations for physical separation between the packer and the pump suggest that a valve is needed immediately after the pressure gauge 324 and the packer 310. Adding a piloted two-way normally open valve 402 and a solenoid control valve 404, it is possible to stop the draw down as soon as flow from the formation has begun and allow the pressure to recompress only the fluid between valve 402 and the formation. Pressure gauge 324 is in this line to accurately measure formation pressure. Since piston displacement is also measured in relation to time and can be correlated with pressure, all of the data necessary for a pressure transient analysis is available at the time valve 404 is closed.

Since the bi-directional pump can be used to repeat tests, one method would be to draw-down in several very small increments of volume, such as 1 cc, and closing valve 402 after each increment and observe whether or not the pressure increases. If not, the next increment would be made until build-up is observed. In each instance the volume to be repressurized is minimized so that repressurization time is minimized.

An alternative method of detecting that flow has begun is to monitor the pressure time response with gauge 324 until a plot of the derivative of the pressure versus time shows that spherical flow has begun (a negative half slope is observed). Valve 402 is then closed, the pump stopped, and pressure build-up is observed. This assures acquisition of valid data with minimum disturbance of the formation.

With the sampling probe 310 in fluid communicating engagement with the formation of interest, the bi-directional piston pump mechanism is operative and selectively controllable to extract formation fluid from the formation, to inject a fluid medium into the formation, to pump the formation fluid so recovered into a sampling tank or to pump the formation fluid into the wellbore. The reversible piston pumping mechanism is capable of recovering a fluid from on-board fluid storage or from the wellbore and injecting it into the formation. This feature is especially important from the standpoint of testing the formation for the effect of a testing fluid such as completion fluid. This invention effectively permits the pumping of fluids of differing viscosity for the purpose of evaluating the characteristics of a formation as determined by the relative viscosity of the formation fluid and the injected fluid. The relative viscosity of the fluids can be of considerable importance from the standpoint of formation productivity, including secondary recovery from the formation of interest. The pumping system of this invention can also achieve pumping to and from the formation or the wellbore. Continuous pumping from the formation into sample tanks may occur for the purpose of clearing the formation interface of debris such as filter cake, fine sand, rocks, etc., thereby permitting the taking of a clean sample for purpose of downhole testing. All of these features can be accomplished without removal of the formation testing instrument from the wellbore. One of the more important features of this invention is the capability of investigating formation pressure, versus time and the capability of correlating it with volume versus time. This capability has not heretofore been available in downhole testing instruments.

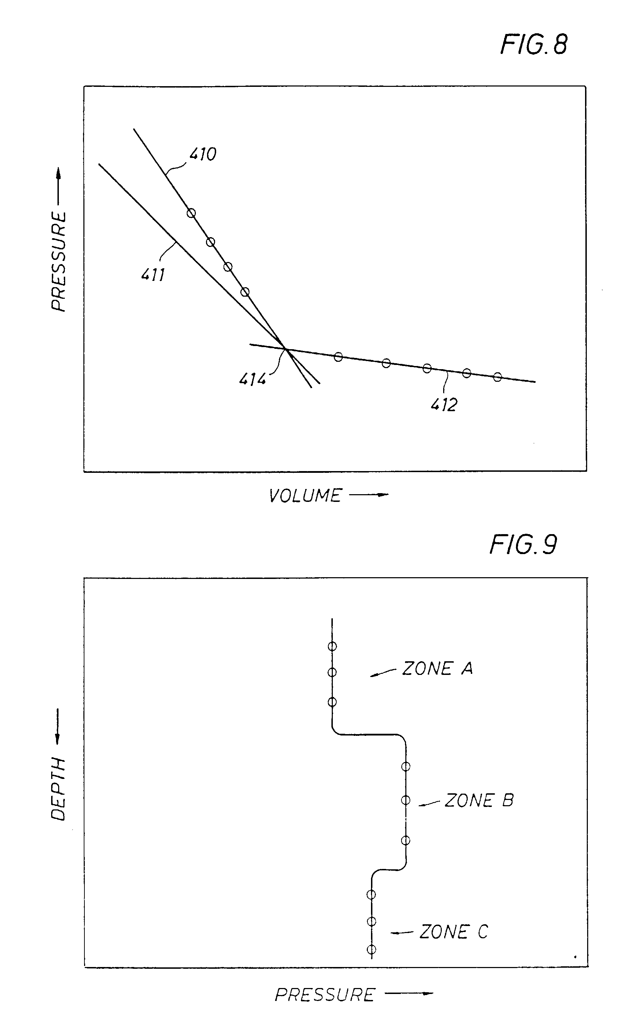

With reference now to Fig. 8, bubble point pressure is determined by confining fluid in a known volume and by observing pressure changes as the volume of the fluid sample is changed. A plot of fluid volume against pressure will indicate fluid phase change from one phase (liquid), as shown by line 410, into two phases (liquid and gas), as shown by line 412. The intersection of two best fit lines, as shown at 414 indicates the bubble point pressure for the sample fluid. Line 411 is representative of variations in fluid compressibility.

Fluid compressibility is calculated as follows:

$$\text{Compressibility} = \frac{1}{V2} * \frac{(V2 - V1)}{(P1 - P2)}$$

where:

V1 = Volume at higher pressure
V2 = Volume at lower pressure
P1 = Higher pressure
P2 = Lower pressure

A typical room temperature value for water is $4.77 \cdot 10^{-7}$ kPa$^{-1}$ ($3.3 \cdot 10^{-6}$ Psi$^{-1}$) and for crude oil is $8.7 \cdot 10^{-6}$ kPa$^{-1}$ ($60 \cdot 10^{-6}$ Psi$^{-1}$), which illustrates that compressibility can also differentiate between oil and water

Referring now to Fig. 9, when bubble point pressure is determined at several depths within a petroleum well, a plot of bubble point pressure against depth is expected to be constant. When this is true, it is likely that tests were conducted on the same fluid. However, if bubble point pressure is different, then different types of fluid were tested and therefore two or more different reservoirs exist. For example, as shown in Fig. 9, bubble point pressure tests identify fluid from three production zones A, B and C.

Referring now to Fig. 10, the graphical representation illustrates three different profile plots of the same subsurface formation in comparison and thus shows how reservoir characterization can be erroneous or incomplete depending on the profile plotting system that is employed. By comparing the curves of Fig. 10 a reservoir having plural zones is identified. The Fig. also indicates how adding fluid compressibility plotted vs formation depth can identify different subsurface production zones which have the same bubble point pressure. Curve "A" is representative of a conventional pressure gradient plot, which substantially defines a straight line, and thus establishes a pressure trend indicating the presence of a single production formation from which the sample fluid is emanating. It is readily seen that, by reliance on curve "A" alone, the actual character of the reservoir does not become recognized. Curve "B" is representative of a bubble point pressure profile which defines a sharply configured offset at 420, thus providing evidence of production fluid emanating from two disconnected subsurface formations. Curve "C" is representative of fluid compressibility (single phase) and indicates by sharply defined offsets at 422 and 424 that three different types of fluids are emanating from the formations defining this particular formation interval.

Fig. 11 is a partial sectional view of one of the cylinder and piston assemblies of the present invention representing a bubble point pressure and fluid compressibility test chamber and which also serves as a piston pumping chamber for the pump-through capability of the downhole PVT multitester instrument. Fig. 11 also provides illustration of hydraulic and electrical circuitry by way of schematics. The supply line or passage 426 for pump chamber 102 is provided with a solenoid operated shutoff valve 428 for entrapment of a finite volume of connate fluid within the pump chamber, thus defining the pump chamber as a test chamber for bubble point pressure and fluid compressibility testing. The test chamber is provided with precision pressure and temperature sensors 430 and 432 respectively to which are coupled electronic signal conductors 434 and 436 respectively that provide signal output "T" and "P" at the surface equipment representing the temperature and pressure of the fluid sample. The precision linear potentiometer 116 is provided with electrical power supply conductors 438 and 440 and includes a position signal output conductor 442 for delivery at the surface equipment of signals representing the position of the piston stem 90 and thus the volume at any point of time of the test chamber 102. For testing, the piston can be precisely located within its cylinder after having drawn in a finite volume of connate fluid as indicated by position signals of the potentiometer. The shutoff valve can then be closed to entrap the sample. After this has been done the pump mechanism may be hydraulically energized to change the volume of the test chamber and thus the volume of the entrapped finite volume of fluid. Observation of the pressure change of the fluid sample during the volume change thereof will be indicative of the bubble point pressure of the fluid. Although a linear type pump cylinder and piston arrangement for bubble point pressure testing has been described above, this invention is not intended to be limited thereby. The multitester instrument may be constructed to establish any suitable variable volume testing chamber the volume of which being sensed in any suitable fashion. Any suitable precision temperature and pressure sensors may be employed within the spirit and scope of this invention.

It has been discovered that bubble point pressure determination plots and bubble point pressure and compressibility profile plots are precise, comprehensive and are more reliable than the conventional pressure gradient plots from the standpoint of reservoir characterization. They have a number of significant advantages in comparison with pressure gradient plots. They are useful in the identification of vertical reservoir continuity in the same reservoir and in fact are more reliable than the conventional pressure gradient method for vertical reservoir continuity. With a pressure gradient profile curve, different types of fluid from different formations may follow the same pressure gradient trend, thereby providing an incorrect indication of a single formation. With the method of the present invention even though different fluids may follow the same pressure gradient trend and have the same bubble point pressure, fluid compressibility will indicate that different fluids are present.

The present method and apparatus may also be employed to identify the drive mechanism of the formation, such as depletion or gas cap drive, for example. If the formation pressure is higher than the bubble point pressure of its fluid, the drive mechanism constitutes a depletion drive of which dissolved gas separates from the fluid and creates the driving influence for flow of the formation fluid toward the well bore. In such case secondary recovery by pressure maintenance is needed to optimize production from this type of formation. If the formation pressure is lower than the bubble point pressure of the fluid being sampled, a gas cap exists, and the flow process driving the formation fluid toward the well bore is due to gas cap expansion. The system of the present invention can also be utilized for identification of low formation pressure, which provides indication that the formation is substantially depleted and thus cannot be effectively produced even under conditions of secondary recovery. The present invention also provides information to define optimum flowing pressure to control gas separation from the connate fluid, thereby enabling the producer to maximize recovery by effective control of production pressure.

In view of the foregoing, it is evident that the present invention is one well adapted to attain all of the objects and features hereinabove set forth, together with other objects and features which are inherent in the apparatus disclosed herein.

As will be readily apparent to those skilled in the art, the present invention may be produced in other specific forms without departing from its spirit or essential characteristics. The present embodiment, is therefore, to be considered as illustrative and not restrictive, the scope of the invention being indicated by the claims rather than the foregoing description, and all changes which come within the meaning and range of the equivalence of the claims are therefore intended to be embraced therein.

## Claims

1. A method for testing a subsurface earth formation (11) having a wellbore (10) defined therein and containing formation fluid, comprising:

   (a) positioning within said wellbore a formation testing instrument (13) having a sampling probe (18) and having a bi-directional piston pump (36) therein being in pumping communication with said sampling probe for selective pumping into and from said subsurface earth formation and having at least one internal sample tank (320);
   (b) projecting said sampling probe from said formation testing instrument into sampling communication with said wellbore at said subsurface earth formation;
   (c) with said bi-directional piston pump forcing a quantity of testing fluid through said sampling probe and into said subsurface earth formation; and
   (d) with said bi-directional piston pump, withdrawing a quantity of said formation fluid from said subsurface earth formation through said sampling probe and into said formation testing instrument.

2. The method of claim 1 wherein signal processing circuitry is provided for processing electronic fluid testing signals, said method further comprising:

   (a) performing at least one formation fluid test on said formation fluid within said testing instrument and developing electronic signals representing the results of said formation fluid test; and
   (b) transmitting said electronic signals from said formation testing instrument to said signal processing circuitry.

3. The method of claim 1 wherein fluid circuits having valves for control thereof are located within said formation testing instrument, said method further comprising:

   after said withdrawing, selectively routing said formation fluid by first valve settings from said subsurface earth formation into said sample tank and by second valve settings routing fluid from said subsurface earth formation through said fluid circuits of said formation testing instrument into said welbore.

4. The method of claim 1, wherein said forcing of said quantity of testing fluid through said sample probe and into said subsurface earth formation comprises:

   (a) with said bi-directional piston pump establishing a predetermined pumping direction, pumping said quantity of testing fluid into said subsurface earth formation by means of said bi-directional piston pump for mixing with said formation fluid to define formation testing fluid treated formation fluid;
   (b) reversing the pumping direction of said bi-directional piston pump from said predetermined pumping direction; and
   (c) pumping said formation testing fluid treated formation fluid from said earth formation into said testing instrument by means of said bi-directional piston pump in said reversed pumping direction thereof.

5. The method of claim 1 wherein said bi-directional piston pump defines at least one pumping chamber (102), said method further comprising:

   said forcing of said formation testing fluid being accomplished by pumping said formation testing fluid from said pumping chamber of said bi-directional piston pump of said testing instrument into said subsurface earth formation.

6. The method of claim 1, including:

   forcing said testing fluid by pumping said testing fluid from said wellbore through said formation testing instrument and into said earth subsurface formation.

7. The method of claim 1, wherein:

   (a) said forcing of said quantity of fluid of step (1) comprises establishing pump directional control of said bi-directional piston pump by selective positioning of instrument valving;
   (b) pumping the fluid from said formation testing instrument through said sampling probe and into said subsurface earth formation; and
   (c) reversing pump directional control of said bi-directional piston pump by selective repositioning of said instrument valving; and
   (d) said withdrawing of step (d) comprising pumping formation fluid from said subsurface earth formation through said sampling probe and into said formation testing instrument by pumping operation of said bi-directional piston pump.

**8.** The method of claim 7, wherein:

(a) after step (d) of claim 7, causing selective positioning of instrument valving ; and
(b) pumping formation fluid through said formation testing instrument to said wellbore for flushing of said debris from said subsurface earth formation.

**9.** The method of claim 7, wherein said formation testing instrument has therein a fluid tank (320) having a formation testing fluid therein, said method including:

(a) after step (d) of claim 7, causing selective positioning of instrument valving; and
(b) with said bi-directional piston pump (36), pumping said formation testing fluid from said fluid tank through said sampling probe for flushing said debris from said subsurface earth formation.

**10.** The method of claim 1, wherein said bi-directional piston pump of said formation testing instrument comprises a double-acting, bi-directional piston pump (36), said method further comprising:

(a) with said double acting bi-directional piston pump, obtaining a sample of said formation fluid;
(b) changing the pressure of said sample of said formation fluid by means of said double-acting, bi-directional piston pump; and
(c) conducting pressure, volume and temperature tests on said pressure changed sample of said formation fluid.

**11.** The method of claim 10, further comprising:

repeating said pressure, volume and temperature tests until a selected fluid sample has been obtained.

**12.** The method of claim 1, wherein a said bi-directional piston pump comprises a double-acting, bi-directional piston pump mechanism being interconnected with said formation testing instrument, said method including:

controllably operating said piston pump mechanism in one selected pumping direction for said forcing step (c) and controllably operating said piston pump mechanism in the opposite selected pumping direction for said withdrawing of step (d).

**13.** The method of claim 12 wherein, said bi-directional piston pump mechanism having a pump body (38) defining a pair of pumping chambers (102, 104) and having an operating piston chamber (80) located intermediate said pumping chambers and further having an operating piston (86) being located for reciprocation within said operating piston chamber (80) and having pumping pistons (94, 96) being fixed to and extending from each side of said operating piston and being respectively received for pumping reciprocation within said pumping chambers, said method further comprising:

(a) with said piston pump mechanism set in a selected pumping direction, controllably reciprocating said operating piston and pumping pistons;
(b) detecting the position of at least one of said operating and pumping pistons within said pump body;
(c) generating electronic position signals representative of said detected position; and
(d) correlating said electronic position signals with time of piston movement to identify the volume of formation fluid pumped by said piston pump mechanism.

**14.** The method of claim 13, including:

electronically varying said volume of formation fluid pumped by said piston pump mechanism.

**15.** The method of claim 13, including:

(a) detecting the temperature of formation fluid being withdrawn from subsurface earth formation; and
(b) correlating said detected formation fluid temperature with said volumetric pumping rate to establish the temperature corrected precision volume of formation fluid pumped by said piston pump mechanism.

**16.** The method of claim 1, including:

(a) detecting the temperature of formation fluid being withdrawn from said subsurface earth formation;
(b) capturing a selected volume of said formation fluid within said formation testing instrument; and
(c) correlating said finite volume of said formation fluid with said detected formation fluid temperature for precision determination of temperature corrected volume thereof.

**17.** The method of claim 16 wherein said formation fluid has a bubble point pressure, said method further comprising:

(a) with said bi-directional piston pump controllably changing the volume of said selected volume of formation fluid;

(b) observing the pressure of said selected volume of formation fluid during said change in volume thereof for determining the bubble point pressure of said formation fluid;

(c) computing the compressibility of said formation fluid; and

(d) utilizing bubble point pressure and compressibility of said formation fluid for determining reservoir characterization of said subsurface earth formation.

18. The method of claim 1, wherein said formation fluid has a bubble point pressure and said bi-directional piston pump (36) defining at least one pump chamber (102) having a pump piston (94) therein and into which said formation fluid is conducted and from which said formation fluid is expelled upon linear movement of the pump piston during pumping activity, said pump chamber (102) further being a bubble point pressure and fluid compressibility test chamber of said formation testing instrument, said formation testing instrument further having means (332, 330, 334, 116) for sensing the temperature of said formation fluid, the pressure of said formation fluid within said test chamber and the volume of said test chamber, and having means for trapping said formation fluid within said test chamber, said method further comprising:

(a) trapping a finite volume of said formation fluid within said test chamber;

(b) comparing the pressure and volume of said trapped formation fluid and providing electronic signals representative thereof;

(c) controllably changing the volume of said trapped formation fluid; (d) observing pressure changes of said trapped formation fluid during volume change thereof;

(e) determining the bubble point pressure of said formation fluid by comparison of said formation fluid pressure, volume, volume change and pressure change; and

(f) utilizing said bubble point pressure of multiple formation fluid tests for reservoir characterization of said subsurface earth formation.

19. A method for flushing debris from a substrate earth formation having a wellbore defined therein and for acquiring uncontaminated samples of formation fluid for testing thereof, said formation fluid having a bubble point pressure, said method further comprising:

(a) positioning within said wellbore a formation testing instrument (13) having a sampling probe (18) and having an on-board fluid tank (320) and having a bi-directional piston pump (36) in selective pumping communication with said sampling probe and said onboard fluid tank;

(b) projecting said sampling probe from said formation testing instrument into sampling communication with said subsurface earth formation;

(c) pumping formation fluid from said subsurface earth formation through said formation testing instrument and into said wellbore until uncontaminated formation fluid is recovered; and

(d) pumping a quantity of said uncontaminated formation fluid into said onboard fluid tank.

20. The method of claim 19, including:

(a) after said pumping step (c) of claim 19, compressing said formation fluid pressure level exceed the bubble point pressure thereof; and

(b) maintaining said pressure level of said formation to prevent phase separation thereof until bubble point analysis is subsequently conducted.

21. The method of claim 19, including:

(a) separating said sampling probe from said selected subsurface formation after said quantity of said uncontaminated formation fluid has been acquired; and

(b) conducting formation fluid tests with said formation testing instrument within said borehole and separated from said selected subsurface earth formation.

22. The method of claim 21, including:

moving said formation testing instrument within said wellbore while conducting said formation fluid tests.

23. Apparatus for conducting pressure, volume and temperature tests of a connate fluid sample from a subsurface earth formation having a wellbore defined therein and for obtaining samples of connate fluid contained within said subsurface earth formation, comprising:

(a) an instrument body structure (32) for positioning at a selected formation depth within said wellbore and having a fluid tank (320) therein containing testing fluid;

(b) a sample probe (310) being laterally extensible from said instrument body structure for fluid sampling engagement with said subsurface earth formation, said sample probe defining a fluid sampling passage (318) for admitting said connate fluid sample from said subsurface earth formation to said instrument and for transferring fluid from said instrument to said subsurface earth formation;

(c) a bi-directional piston pump (36) being located within said instrument body structure and having a pump cylinder (80) and a piston (86) being movable within said pump cylinder; and

(d) means (350, 352, 360, 362) selectively controlling the pumping direction of said piston pump mechanism for selective pumping of said testing fluid through said sampling probe into said surface earth formation and pumping of formation fluid from said subsurface earth formation through said sampling probe and into said body structure.

24. The apparatus of claim 23, wherein:

(a) said instrument body defines a testing chamber (102) therein adapted to contain a finite volume of said connate fluid therein;

(b) means (330, 332) for sensing the formation pressure and temperature of said connate fluid;

(c) means (116) for changing the volume and sensing the volume change of said finite volume of said connate fluid within said testing chamber; and

(d) means (324) for sensing pressure changes of said finite volume of said connate fluid during said changing of the volume thereof for establishing the bubble pressure point of said connate fluid.

25. The apparatus of claim 24, including:

means (116, 324) associated with said testing chamber for determining the compressibility of said connate fluid within said testing chamber.

26. The apparatus of claim 24, wherein:

said piston pump mechanism defines at least one pump chamber mechanism (80) having a piston (86) reciprocable therein for pumping activity, said pump chamber defining said testing chamber (104).

27. The apparatus of claim 26, wherein said piston (86) defines said means for changing the volume of said finite volume of said connate fluid.

28. The apparatus of claim 27, including:

a linear potentiometer (116) within said instrument body (32) and being disposed in movement sensing relation with said piston, said linear potentiometer providing electronic position indicating signals reflecting said finite volume of said connate fluid within said testing chamber and changing of said finite volume of said connate fluid within said testing chamber.

29. The apparatus of claim 26, including:

(a) a fluid passage (426) being defined by said instrument body and being in communication with said fluid sampling passage of said sampling probe and with said pump chamber (102); and

(b) an isolation means (428) being disposed in communication with said fluid sampling passage and being selectively operated for trapping said finite volume of said connate fluid within said test chamber for bubble point pressure testing thereof.

30. The apparatus of claim 26, including:

(a) a temperature sensor (432) being coupled with said pump chamber (102) for sensing the formation temperature of said connate fluid within said testing chamber; and

(b) a pressure sensor (430) being coupled with said pump chamber (102) for sensing the formation pressure of said connate fluid within said pump chamber (102) and for sensing said pressure changes of said finite volume of said connate fluid during said volume changing thereof.

31. The apparatus of claim 24, including:

(a) a movable wall (86) defining a portion of said testing chamber; and

(b) a potentiometer (116) being operatively movable by said movable wall and providing electronic output signals reflecting positions of said movable wall and thus the volume of said testing chamber for determination of said finite volume and volume changing of said connate fluid within said testing chamber.

32. The apparatus of claim 23, further comprising:

(a) position sensing means (116) for sensing the position of said bi-directional piston pump (36) and generating electronic piston position signals; and

(b) means (22) for receiving and processing said electronic piston position signals for identification of the volume of connate fluid pumped by said bi-directional piston pump.

**33.** The apparatus of claim 32, further comprising:

(a) means (330, 332) for detecting the pressure and temperature of formation fluid entering said formation testing instrument from said subsurface earth formation and generating electronic pressure and temperature signals relating thereto; and

(b) means (22) for processing said electronic pressure and temperature signals in correlation with said electronic piston position signals for derivation of pressure and temperature corrected volumetric displacement of said connate fluid by said piston pump mechanism.

**34.** The apparatus of claim 23, wherein:

(a) said bi-directional piston pump mechanism (19) being a bi-directional piston pump (36) having opposed pumping chambers (102, 104);

(b) said means (168, 170) for selectively controlling the pumping direction of said piston pump being a hydraulic fluid pumping circuit having check valves permitting unidirectional flow of fluid to and from said pumping chambers; and

(c) a reversing valve (240, 242) coupled in said hydraulic fluid pumping circuit for selecting the direction of fluid flow to and from said check valves.

**35.** The apparatus of claim 32, wherein said bi-directional piston pump defines a pair of pump cylinders each having a pumping piston movably located therein and said position sensing means (116) comprises:

(a) a position indicator rod (114) extending from one of said pumping pistons; and

(b) a linear movement potentiometer receiving said position indicator rod therein and generating said electronic piston position signals reflecting the position of said position indicator rod relative to said linear movement potentiometer.

**36.** The apparatus of claim 23, wherein said bi-directional piston pump mechanism comprises:

(a) a pump body (36) defining a pair of pumping chambers (102, 104) and an operating piston chamber (80) disposed intermediate said pumping chambers;

(b) an operating piston (86) located for hydraulically energized reciprocation within said operating piston chamber (80);

(c) a pair of pumping pistons (94, 96) fixed to and extending from opposed sides of said operating piston (86) and being received for pumping reciprocation within respective pumping chambers (102, 104);

(d) a hydraulic pump operation circuit (Fig. 5) for inducing operating reciprocation of said operation piston within said operating piston chamber;

(e) a connate fluid circuit (Fig. 6) being located within said instrument body structure and being in communication with said pumping chambers and receiving formation fluid from said pumping chambers upon reciprocation of said pumping pistons; and

(f) directional control means (Fig. 4) for selectively directing the flow of formation fluid within said formation fluid circuit to said subsurface earth formation or from said subsurface earth formation.

**37.** The apparatus of claim 36, wherein said formation fluid circuit (Fig. 6) includes a fluid source within said instrument body and a fluid receptacle within said instrument body and said directional control means comprises:

(a) a flow reversing valve (168) within said formation fluid circuit having a first flow controlling position directing formation fluid flow from said subsurface earth formation to said pumping chambers (102, 104) and from said pumping chambers to a sample receiver (320), said flow reversing valve having a second flow controlling position directing fluid from said fluid source to said pumping chambers and from said pumping chambers to said subsurface earth formation; and

(b) means (240) for controllably positioning said flow reversing valve at said first or second flow controlling positions.

**38.** The apparatus of claim 37, wherein said means for controllably positioning said flow reversing valve comprises:

(a) a pilot pressure supply (236) being operatively coupled to said flow reversing valve; and

(b) a control valve (240) selectively controlling application of pilot pressure from said pilot pressure supply to said reversing valve for selected operating thereof to said first and second flow controlling positions.

**39.** The apparatus of claim 37, wherein said flow reversing valve (168) comprises:

(a) a valve seat sleeve (192) being retained in sealed relation within said instrument body structures and forming a plurality of internal sealing surface segments, said valve seat sleeve having spaced annual external fluid conducting grooves disposed in communication with respective hydraulic fluid and formation fluid flow passages of said instrument body structure, said valve seat sleeve further having a plurality of internal annual fluid conducting grooves (214) separating said internal sealing surface segments;

(b) a valve spool (218) being movably positioned in sealed relation within said valve seat sleeve and defining spaced external annular fluid conducting grooves in selective communication with said annular internal fluid conducting grooves of said valve seat sleeve and having internal fluid conducting passages; and

(c) means (328) selectively hydraulically shifting said valve spool from said first flow controlling position to said second flow controlling position.

40. The apparatus of claim 39, wherein said apparatus includes at least one sample storage tank (320) located within said instrument body and said valve spool includes:

first and second check valve controlled passages (343, 345) each permitting unidirectional flow of formation fluid and being selectively operable depending upon the position of said valve spool to permit pumped flow of formation fluid from or to said subsurface earth formation and from or to said borehole or sample storage tank.

41. The apparatus of claim 39, including:

selecting means (170) within said instrument body for directing the pumped flow of formation fluid through said flow reversing valve to said wellbore or to said sample storage tank.

42. The apparatus of claim 41, wherein said selecting means comprises:

(a) a flow passage (302) connecting said passages of flow reversing valve with said wellbore and said storage tank; and

(b) at least one control valve (168) for selectively controlling the flow of formation fluid from said reversing valve to said wellbore or to said sample storage tank.

43. A downhole formation testing instrument (13) comprising:

(a) an instrument body (32) adapted for positioning at formation depth within a wellbore and defining a pump chamber (36) a fluid sampling and pumping passage (106) in communication with said pump chamber;

(b) a sample probe (18) cooperatively arranged on and laterally extensible from said instrument body structure for fluid sampling engagement with a selected subsurface earth formation, said sample probe defining a fluid sample passage (106) for admitting connate fluid from said subsurface earth formation;

(c) packer means (25) isolating said sampling passage from wellbore pressure and ensuring communication of connate fluid into said sample probe substantially at formation pressure;

(d) a bi-directional piston pump mechanism (36) being located within said instrument body structure and defining at least one pumping chamber (80) having fluid communication with said fluid sampling passage, said pumping chamber further defining a testing chamber for said connate fluid, said piston pumping mechanism having a piston (86) reciprocable within said pumping chamber, said piston defining a movable wall of said testing chamber;

(e) means (116) for sensing piston movement and positioning within said pumping chamber and thus sensing finite volume and volume change of said testing chamber and the connate fluid contained therein;

(f) means (320) for trapping a finite volume of connate fluid within said testing chamber;

(g) said piston (86) being selectively movable within said pumping chamber for changing said finite volume of connate fluid contained within said testing chamber for bubble point pressure determination thereof;

(h) means (432) for sensing the temperature of connate fluid within said testing chamber; and

(i) means for sensing the initial pressure (430) of connate fluid within said testing chamber and for sensing pressure change of said connate fluid during volume change thereof.

44. The downhole formation testing instrument of claim 43, including:

a potentiometer within said instrument body and being disposed in movement sensing relation with said piston, said potentiometer providing electronic position indicating signals reflecting said finite volume of said connate fluid within said testing chamber and changing of said finite volume of said connate fluid within said testing chamber.

45. The downhole formation testing instrument of claim 43, including:

(a) a fluid passage (106) being defined by said instrument body and being in communication with said fluid sampling passage of said sampling probe and with said pump chamber; and
(b) isolation means (25) being disposed in communication with said fluid sampling passage and being selectively operated for trapping said finite volume of said connate fluid within said test chamber for bubble point pressure testing thereof.

**46.** The downhole formation testing instrument of claim 43, including:

(a) a temperature sensor (432) being coupled with said pump chamber (104) for sensing the formation temperature of said connate fluid within said testing chamber; and
(b) a pressure sensor (430) being coupled with said pump chamber (104) for sensing the formation pressure of said connate fluid within said pump chamber and for sensing said pressure changes of said finite volume of said connate fluid during said volume changing thereof.

**47.** The downhole formation testing instrument of claim 43, including:

(a) a movable wall (86) defining a portion of said testing chamber;
(b) a potentiometer (116) being operatively movable by said movable wall and providing electronic output signals reflecting positions of said movable wall and thus the volume of said testing chamber for determination of said finite volume and volume changing of said connate fluid within said testing chamber (104).

**Patentansprüche**

**1.** Verfahren zum Untersuchen einer unterirdischen Bodenformation (11), in der ein Bohrloch (10) bestimmt ist, und die Formationsfluid enthält, umfassend:

a) das Anordnen eines Formationsprüfgeräts (13) in dem Bohrloch, wobei das Formationsprüfgerät aufweist: einen Probenentnehmer (18); eine zweiseitige Kolbenpumpe (36) im Gerät, die in Pumpverbindung mit dem Probenentnehmer steht und ausgewählt in die unterirdische Bodenformation hineinpumpt bzw. aus ihr heraus; und zumindest einen inneren Probentank (320);
b) das Ausfahren des Probenentnehmers aus dem Formationsprüfgerät in eine Prüfverbindung mit dem Bohrloch und der unterirdischen Bodenformation;
c) das Drücken eines Quantums Prüffluid mit der zweiseitigen Kolbenpumpe durch den Probenentnehmer und in die unterirdische Bodenformation; und
d) das Herausziehen eines Quantums Formationsfluid mit der zweiseitigen Kolbenpumpe aus der unterirdischen Bodenformation durch den Probenentnehmer und in das Formationsprüfgerät.

**2.** Verfahren nach Anspruch 1, wobei Signalverarbeitungsschaltungen zum Verarbeiten elektronischer Fluidprüfsignale bereitgestellt sind, zudem umfassend:

a) das Ausführen von mindestens einer Formationsfluidprüfung auf dem Formationsfluid innerhalb des Prüfgeräts und das Ausbilden elektronischer Signale, die die Ergebnisse der Formationsfluidprüfung darstellen; und
b) das Übertragen der elektronischen Signale aus dem Formationsprüfgerät zu den Signalverarbeitungsschaltungen.

**3.** Verfahren nach Anspruch 1, wobei innerhalb des Formationsprüfgeräts Fluidkreise angeordnet sind, die Ventile zum Steuern der Fluidkreise aufweisen, zudem umfassend:

nach dem Herausziehen das ausgewählte Weiterleiten des Formationsfluids durch erste Ventileinstellungen aus der unterirdischen Bodenformation in den Probentank und das Weiterleiten des Fluids durch zweite Ventileinstellungen aus der unterirdischen Bodenformation durch die Fluidkreise des Formationsprüfgeräts in das Bohrloch.

**4.** Verfahren nach Anspruch 1, wobei das Drücken des Quantums Prüffluid durch den Probenentnehmer und in die unterirdische Bodenformation umfaßt:

a) Festlegen einer vorbestimmten Pumprichtung mit der zweiseitigen Kolbenpumpe und Pumpen des Quantums Prüffluid in die unterirdische Bodenformation mit Hilfe der zweiseitigen Kolbenpumpe, um das Prüffluid mit dem Formationsfluid zu vermischen und ein Formationsfluid zu bestimmen, das mit Formationsprüffluid behandelt ist;
b) Umkehren der Pumprichtung der zweiseitigen Kolbenpumpe gegen die vorbestimmte Pumprichtung; und
c) Pumpen des mit Formationsprüffluid behandelten Formationsfluids aus der Bodenformation in das Prüfgerät mit Hilfe der zweiseitigen Kolbenpumpe in ihrer umgekehrten Pumprichtung.

5. Verfahren nach Anspruch 1, wobei die zweiseitige Kolbenpumpe zumindest eine Pumpkammer (102) bestimmt, und das Verfahren zudem umfaßt:

daß das Drücken des Formationsprüffluids erreicht wird durch das Pumpen des Formationsprüffluids aus der Pumpkamner der zweiseitigen Kolbenpumpe des Prüfgeräts in die unterirdische Bodenformation.

6. Verfahren nach Anspruch 1, umfassend:

das Drücken des Prüffluids durch das Pumpen des Prüffluids aus dem Bohrloch durch das Formationsprüfgerät und in die unterirdische Bodenformation.

7. Verfahren nach Anspruch 1, wobei:

a) das Drücken des Quantums Fluid aus Schritt 1) das Festlegen der Pumprichtungssteuerung der zweiseitigen Kolbenpumpe durch das ausgewählte Anordnen der Geräteventile umfaßt;
b) das Pumpen des Fluids aus dem Formationsprüfgerät durch den Probenentnehmer und in die unterirdische Bodenformation erfolgt;
c) das Umkehren der Pumprichtungssteuerung der zweiseitigen Kolbenpumpe durch das ausgewählte Umordnen der Geräteventile erfolgt; und
d) das Herausziehen nach Schritt d) das Pumpen des Formationsfluids aus der unterirdischen Bodenformation durch den Probenentnehmer und in das Formationsprüfgerät durch einen Pumpvorgang der zweiseitigen Kolbenpumpe umfaßt.

8. Verfahren nach Anspruch 7, wobei:

a) nach dem Schritt d) in Anspruch 7 das ausgewählte Anordnen der Geräteventile erfolgt; und
b) Formationsfluid durch das Formationsprüfgerät in das Bohrloch gepumpt wird, um das Bohrmehl aus der unterirdischen Bodenformation auszuspülen.

9. Verfahren nach Anspruch 7, wobei das Formationsprüfgerät in sich einen Fluidtank (320) hat, in dem sich Formationsprüffluid befindet, und das Verfahren umfaßt:

a) nach dem Schritt d) aus Anspruch 7 das ausgewählte Anordnen der Geräteventile; und
b) das Pumpen des Formationsprüffluids mit der zweiseitigen Kolbenpumpe (36) aus dem Fluidtank durch den Probenentnehmer, um das Bohrmehl aus der unterirdischen Bodenformation auszuspülen.

10. Verfahren nach Anspruch 1, wobei die zweiseitige Kolbenpumpe des Formationsprüfgeräts eine doppeltwirkend zweiseitige Kolbenpumpe (36) enthält und das Verfahren ferner umfaßt:

a) das Erfassen einer Probe des Formationsfluids mit der doppeltwirkenden zweiseitigen Kolbenpumpe;
b) das Ändern des Drucks der Formationsfluidprobe mit Hilfe der doppeltwirkenden zweiseitigen Kolbenpumpe; und
c) das Ausführen von Druck-, Volumen- und Temperaturuntersuchungen an der druckveränderten Formationsfluidprobe.

11. Verfahren nach Anspruch 10, zudem umfassend:

das Wiederholen der Druck-, Volumen- und Temperaturuntersuchungen, bis man eine ausgewählte Fluidprobe erhalten hat.

12. Verfahren nach Anspruch 1, wobei die zweiseitige Kolbenpumpe einen doppeltwirkenden zweiseitigen Kolbenpumpenmechanismus enthält, der mit dem Formationsprüfgerät verbunden ist, und das Verfahren umfaßt:

das steuerbare Betreiben des Kolbenpumpenmechanismus in eine ausgewählte Pumprichtung für den Drückschritt c) und das steuerbare Betreiben des Kolbenpumpenmechanismus in die entgegengesetzte ausgewählte Pumprichtung für den Herausziehschritt d).

13. Verfahren nach Anspruch 12, wobei der zweiseitige Kolbenpumpenmechanismus einen Pumpenkörper (38) aufweist, der ein Paar Pumpkammern (102, 104) bestimmt, und eine Betätigungskolbenkammer (80), die zwischen den Pumpkammern angeordnet ist, und zudem einen Betätigungskolben (86), der für eine Hin- und Herbewegung in der Betätigungskolbenkammer (80) angeordnet ist und Pumpkolben (94, 96) hat, die an jeder Seite des Betätigungskolbens befestigt sind und davon ausgehen und jeweils für eine pumpende Hin- und Herbewegung in den Pumpkammern aufgenommen sind, und das Verfahren zudem umfaßt:

a) das steuerbare Hin- und Herbewegen des Betätigungskolbens und der Pumpkolben, wobei der Kolbenpumpenmechanismus auf eine ausgewählte Pumprichtung eingestellt ist;
b) das Erfassen der Lage zumindest eines Kolbens unter dem Betätigungskolben und den Pumpkolben innerhalb des Pumpenkörpers;
c) das Erzeugen elektronischer Lagesignale, die die erfaßte Position darstellen; und
d) das Korrelieren der elektronischen Lagesignale mit der Zeit der Kolbenbewegung, um das Volumen des Formationsfluids zu erkennen, das der Kolbenpumpenmechanismus pumpt.

14. Verfahren nach Anspruch 13, umfassend:

das elektronische Verändern des Formationsfluidvolumens, das der Kolbenpumpenmechanismus pumpt.

15. Verfahren nach Anspruch 13, umfassend:

a) das Erfassen der Temperatur des Formationsfluids, das aus der unterirdischen Bodenformation herausgezogen wird; und
b) das Korrelieren der erfaßten Formationsfluidtemperatur mit der volumetrischen Pumprate, um das temperaturkorrigierte Präzisionsvolumen des Formationsfluids zu bestimmen, das der Kolbenpumpenmechanismus pumpt.

16. Verfahren nach Anspruch 1, umfassend:

a) das Erfassen der Temperatur des Formationsfluids, das aus der unterirdischen Bodenformation herausgezogen wird;
b) das Einfangen eines ausgewählten Volumens des Formationsfluids innerhalb des Formationsprüfgeräts; und
c) das Korrelieren des endlichen Formationsfluidvolumens mit der erfaßten Formationsfluidtemperatur für eine präzise Bestimmung des temperaturkorrigierten Volumens des Formationsfluids.

17. Verfahren nach Anspruch 16, wobei das Formationsfluid einen Blasenpunktdruck hat, und das Verfahren weiterhin umfaßt:

a) das steuerbare Verändern des Volumens des ausgewählten Formationsfluidvolumens mit der zweiseitigen Kolbenpumpe;
b) das Beobachten des Drucks des ausgewählten Formationsfluidvolumens während seiner Volumenänderung, um den Blasenpunktdruck des Formationsfluids zu bestimmen;
c) das Berechnen der Verdichtbarkeit des Formationsfluids; und
d) das Verwenden des Blasenpunktdrucks und der Verdichtbarkeit des Formationsfluids zum Bestimmen der Lagerstätteneigenschaften der unterirdischen Bodenformation.

18. Verfahren nach Anspruch 1, wobei das Formationsfluid einen Blasenpunktdruck hat, und die zweiseitige Kolbenpumpe (36) zumindest eine Pumpkammer (102) bestimmt, in der sich ein Pumpkolben (94) befindet und in die das Formationsfluid geleitet wird und aus der das Formationsfluid durch eine geradlinige Bewegung des Pumpkolbens während des Pumpvorgangs ausgestoßen wird, und die Pumpkammer (102) zudem eine Blasenpunktdruck- und Fluidverdichtbarkeits-Prüfkammer des Formationsprüfgeräts ist, und das Formationsprüfgerät zudem Vorrichtungen (332, 330, 334, 116) hat, die die Temperatur des Formationsfluids, den Druck des Formationsfluids in der Prüfkammer und das Volumen der Prüfkammer erfassen, und das Formationsprüfgerät eine Vorrichtung besitzt, die das Formationsfluid in der Prüfkammer festhält, und das Verfahren auch umfaßt:

a) das Festhalten eines begrenzten Formationsfluidvolumens in der Prüfkammer;
b) das Vergleichen des Drucks und des Volumens des festgehaltenen Formationsfluids und das Bereitstellen elektronischer Signale, die diese Größen darstellen;
c) das steuerbare Verändern des Volumens des festgehaltenen Formationsfluids;
d) das Beobachten von Druckänderungen des festgehaltenen Formationsfluids während der Volumenänderung des Formationsfluids;
e) das Bestimmen des Blasenpunktdrucks des Formationsfluids durch den Vergleich von Formationsfluiddruck, Volumen, Volumenänderung und Druckänderung; und
f) das Verwenden des Blasenpunktdrucks aus zahlreichen Formationsfluiduntersuchungen zur Lagerstättenkennzeichnung der unterirdischen Bodenformation.

19. Verfahren zum Ausspülen von Bohrmehl aus einer unterirdischen Bodenformation, in der ein Bohrloch bestimmt ist, und zum Gewinnen unverschmutzter Proben des Formationsfluids, um sie zu untersuchen, wobei das Formationsfluid einen Blasenpunktdruck hat, und das Verfahren ferner umfaßt:

a) das Positionieren eines Formationsprüfgeräts (13) in dem Bohrloch, wobei das Formationsprüfgerät aufweist: einen Probenentnehmer (18); einen eingebauten Fluidtank (320); und eine zweiseitige Kolbenpumpe (36), die in ausgewählter Pumpverbindung mit dem Probenentnehmer und dem eingebauten Fluidtank steht;
b) das Ausfahren des Probenentnehmers aus dem Formationsprüfgerät in eine Prüfverbindung mit der unterirdischen Bodenformation;
c) das Pumpen von Formationsfluid aus der unterirdischen Bodenformation durch das Formationsprüfgerät und in das Bohrloch, bis man unverschmutztes Formationsfluid erhält; und

d) das Pumpen eines Quantums des unverschmutzten Formationsfluids in den eingebauten Fluidtank.

**20.** Verfahren nach Anspruch 19, umfassend:

a) nach dem Pumpschritt c) aus Anspruch 19, das Verdichten des Formationsfluids, so daß die Druckhöhe den Blasenpunktdruck des Formationsfluids übersteigt; und

b) das Beibehalten der Druckhöhe des Formationsfluids, um eine Phasentrennung des Formationsfluids zu verhindern, bis die nachfolgende Blasenpunktuntersuchung ausgeführt wird.

**21.** Verfahren nach Anspruch 19, umfassend:

a) das Trennen des Probenentnehmers von der ausgewählten unterirdischen Formation, nachdem das Quantum unverschmutzten Formationsfluids gewonnen worden ist; und

b) das Ausführen von Formationsfluiduntersuchungen mit dem Formationsprüfgerät innerhalb des Bohrlochs und getrennt von der ausgewählten unterirdischen Bodenformation.

**22.** Verfahren nach Anspruch 21, umfassend:

das Bewegen des Formationsprüfgeräts innerhalb des Bohrlochs, während Formationsfluiduntersuchungen ausgeführt werden.

**23.** Einrichtung zum Ausführen von Druck-, Volumen- und Temperaturuntersuchungen an einer fossilen Fluidprobe aus einer unterirdischen Bodenformation, in der ein Bohrloch bestimmt ist, und zum Gewinnen von Proben eines fossilen Fluids, das in der unterirdischen Bodenformation enthalten ist, umfassend:

a) eine Gerätekörperanordnung (32), die auf einer ausgewählten Formationstiefe innerhalb des Bohrlochs angeordnet wird und einen Fluidtank (320) in der Anordnung hat, der Prüffluid enthält;

b) einen Probenentnehmer (310), der seitlich aus der Gerätekörperanordnung ausfahrbar ist und zum Gewinnen von Fluidproben in die unterirdische Bodenformation eingreift, wobei der Probenentnehmer einen Fluidprobendurchgang (318) bestimmt, um die fossile Fluidprobe aus der unterirdischen Bodenformation in das Gerät aufzunehmen und zum Übertragen von Fluid aus dem Gerät in die unterirdische Bodenformation;

c) eine zweiseitige Kolbenpumpe (36), die in der Gerätekörperanordnung untergebracht ist und einen Pumpenzylinder (80) und einen Kolben (86) besitzt, der sich in dem Pumpenzylinder bewegen kann, und

d) Vorrichtungen (350, 352, 360, 362), die ausgewählt die Pumprichtung des Kolbenpumpenmechanismus steuern und das Prüffluid ausgewählt durch den Probenentnehmer in die unterirdische Bodenformation pumpen, und das Formationsfluid aus der unterirdischen Bodenformation durch den Probenentnehmer in die Körperanordnung pumpen.

**24.** Einrichtung nach Anspruch 23, worin:

a) der Gerätekörper eine Prüfkammer (102) darin bestimmt, die dazu eingerichtet ist, ein begrenztes Volumen des fossilen Fluids in sich zu enthalten;

b) Vorrichtungen (330, 332) zum Erfassen des Formationsdrucks und der Temperatur des fossilen Fluids enthalten sind;

c) eine Vorrichtung (116) zum Verändern des Volumens und zum Erfassen der Volumenänderung des begrenzten Volumens des fossilen Fluids in der Prüfkammer enthalten ist; und

d) eine Vorrichtung (324) zum Erfassen von Druckänderungen des begrenzten Volumens des fossilen Fluids während der Volumenänderung des Fluids enthalten ist, um den Blasenpunktdruck des fossilen Fluids festzulegen.

**25.** Einrichtung nach Anspruch 24, umfassend:

Vorrichtungen (116, 324), die zur Prüfkammer gehören und die Verdichtbarkeit des fossilen Fluids in der Prüfkammer feststellen.

**26.** Einrichtung nach Anspruch 24, worin:

der Kolbenpumpenmechanismus zumindest einen Pumpenkammermechanismus (80) bestimmt, der einen Kolben (86) hat, der sich für den Pumpvorgang in der Pumpkammer Hin- und Her bewegen kann, und die Pumpkammer die Prüfkammer (104) bestimmt.

**27.** Einrichtung nach Anspruch 26, wobei der Kolben (86) die Vorrichtung zum Verändern des Volumens des begrenzten Volumens des fossilen Fluids bestimmt.

**28.** Einrichtung nach Anspruch 27, umfassend:

ein gerades Potentiometer (116) innerhalb des Gerätekörpers (32), das so angeordnet ist, daß es die Kolbenbewegungen erfaßt, wobei das gerade Potentiometer elektronische Positionsanzeigesignale liefert, die das begrenzte Volumen des fossilen Fluids innerhalb der Prüfkammer und eine Veränderung des begrenzten Volumens des fossilen Fluids innerhalb der Prüfkammer widerspiegeln.

29. Einrichtung nach Anspruch 26, umfassend:

a) einen Fluiddurchgang (426), der durch den Gerätekörper bestimmt und mit dem Fluidprobendurchgang des Probenentnehmers und mit der Pumpkammer (102) verbunden ist; und
b) eine Isoliervorrichtung (428), die verbunden mit dem Fluidprobendurchgang angeordnet ist und ausgewählt betrieben wird, um das begrenzte Volumen des fossilen Fluids für die Blasenpunktdruck-Bestimmung des Fluids in der Prüfkammer einzuschließen.

30. Einrichtung nach Anspruch 26, umfassend:

a) einen Temperatursensor (432), der mit der Pumpkammer (102) verbunden ist und die Formationstemperatur des fossilen Fluids in der Prüfkammer erfaßt; und
b) einen Drucksensor (430), der mit der Pumpkamner (102) verbunden ist und den Formationsdruck des fossilen Fluids in der Prüfkammer (102) und die Druckänderungen des begrenzten Volumens des fossilen Fluids während der Volumenänderung des Fluids erfaßt.

31. Einrichtung nach Anspruch 24, umfassend:

a) eine bewegliche Wand (86), die einen Abschnitt der Prüfkammer bestimmt; und
b) ein Potentiometer (116), das von der beweglichen Wand funktional betätigt werden kann und elektronische Ausgangssignale liefert, die die Stellungen der beweglichen Wand widerspiegeln und damit das Volumen der Prüfkammer, um das begrenzte Volumen und die Volumenänderung des fossilen Fluids innerhalb der Prüfkammer zu bestimmen.

32. Einrichtung nach Anspruch 23, zudem umfassend:

a) eine Lageerfassungsvorrichtung (116), die die Stellung der zweiseitigen Kolbenpumpe (36) erfaßt und elektronische Kolbenstellungssignale erzeugt; und
b) Vorrichtungen (22) zum Empfangen und Verarbeiten der elektronischen Kolbenstellungssignale, um das Volumen des fossilen Fluids zu erkennen, das die zweiseitige Kolbenpumpe pumpt.

33. Einrichtung nach Anspruch 32, zudem umfassend:

a) Vorrichtungen (330, 332), die den Druck und die Temperatur des Formationsfluids erfassen, das aus der unterirdischen Bodenformation in das Formationsprüfgerät eintritt, und elektronische Druck- und Temperatursignale erzeugen, die sich darauf beziehen; und
b) eine Vorrichtung (22), die die elektronischen Druck- und Temperatursignale in Korrelation mit den elektronischen Kolbenstellungssignalen verarbeitet, um das druck- und temperaturkorrigierte Saugvolumen des fossilen Fluids abzuleiten, das der Kolbenpumpenmechanismus liefert.

34. Einrichtung nach Anspruch 23, wobei:

a) der zweiseitige Kolbenpumpenmechanismus (19) eine zweiseitige Kolbenpumpe (36) ist, die gegenüberliegende Pumpkammern (102, 104) hat;
b) die Vorrichtungen (168, 170), die die Pumprichtung der Kolbenpumpe ausgewählt steuern, ein hydraulischer Fluidpumpkreis sind, der Rückschlagventile hat, die eine Fluidströmung zu und von den Pumpkammern in einer Richtung erlauben; und
c) ein Umschaltventil (240, 242), das in den hydraulischen Fluidpumpkreis geschaltet ist, um die Richtung der Fluidströmung zu und von den Rückschlagventilen auszuwählen.

35. Einrichtung nach Anspruch 32, wobei die zweiseitige Kolbenpumpe ein Paar Pumpzylinder bestimmt, in denen jeweils ein Pumpkolben beweglich angeordnet ist, und die Lageerfassungsvorrichtung (116) umfaßt:

a) eine Lagegeberstange (114), die von einem der Pumpkolben ausgeht; und
b) ein Potentiometer mit geradliniger Bewegung, das die Lagegeberstange in sich aufnimmt und die elektronischen Kolbenstellungssignale erzeugt, die die Position der Lagegeberstange relativ zum Potentiometer mit geradliniger Bewegung widerspiegeln.

36. Einrichtung nach Anspruch 23, wobei der zweiseitige Kolbenpumpenmechanismus umfaßt:

a) einen Pumpenkörper (36), der ein Paar Pumpkammern (102, 104) bestimmt, und eine Betätigungskolbenkammer (80), die zwischen den Pumpkammern angeordnet ist;
b) einen Betätigungskolben (86), der für eine hydraulisch angetriebene Hin- und Herbewegung in der Betätigungskolbenkammer (80) angeordnet ist;

c) ein Paar Pumpkolben (94, 96), die an entgegengesetzten Seiten des Betätigungskolbens (86) befestigt sind und davon ausgehen und für eine pumpende Hin- und Herbewegung in den jeweiligen Pumpkammern (102, 104) aufgenommen sind;

d) einen hydraulischen Pumpenbetätigungskreis, siehe Fig. 5, der eine Hin- und Her gehende Betätigungsbewegung des Betätigungskolbens in der Betätigungskolbenkammer veranlaßt;

e) einen Kreis für fossiles Fluid, siehe Fig. 6, der in der Gerätekörperanordnung untergebracht und mit den Pumpkammern verbunden ist und bei der Hin- und Herbewegung der Pumpkolben Formationsfluid aus den Pumpkammern aufnimmt; und

f) eine Richtungssteuervorrichtung, siehe Fig. 4, die ausgewählt die Richtung der Formationsfluidströmung im Formationsfluidkreis zur unterirdischen Bodenformation hin oder von der unterirdischen Bodenformation weg anweist.

37. Einrichtung nach Anspruch 36, wobei der Formationsfluidkreis, siehe Fig. 6, eine Fluidquelle innerhalb des Gerätekörpers enthält und eine Fluidaufnahme im Gerätekörper, und die Richtungssteuervorrichtung umfaßt:

a) ein Strömungsumkehrventil (168) innerhalb des Formationsfluidkreises, das eine erste Strömungssteuerstellung hat, die Formationsfluid aus der unterirdischen Bodenformation in die Pumpkammern (102, 104) leitet, und von den Pumpkammern zu einem Probenaufnehmer (320), wobei das Strömungsumkehrventil eine zweite Strömungssteuerstellung hat, die Fluid aus der Fluidquelle zu den Pumpkammern und von den Pumpkammern zu der unterirdischen Bodenformation leitet; und

b) eine Vorrichtung (240), die das Strömungsumkehrventil gesteuert in die erste oder die zweite Strömungssteuerstellung bringt.

38. Einrichtung nach Anspruch 37, wobei die Vorrichtung, die das Strömungsumkehrventil gesteuert in Stellung bringt, umfaßt:

a) eine Steuerdruckzufuhr (236), die funktional mit dem Strömungsumkehrventil verbunden ist; und

b) ein Steuerventil (240), das ausgewählt die Ausübung des Steuerdrucks aus der Steuerdruckzufuhr auf das Umkehrventil steuert, um das Umkehrventil ausgewählt in die erste und die zweite Strömungssteuerstellung zu bringen.

39. Einrichtung nach Anspruch 37, wobei das Strömungsumkehrventil (168) umfaßt:

a) eine Ventilsitzhülse (192), die innerhalb der Gerätekörperanordnungen dichtend festgehalten wird und eine Anzahl innerer Dichtflächenabschnitte bildet, wobei die Ventilsitzhülse äußere beabstandete ringförmige Fluidleitungsnuten aufweist, die verbunden mit jeweiligen Hydraulikfluid- und Formationsfluid-Strömungsdurchgängen der Gerätekörperanordnung angeordnet sind, und die Ventilsitzhülse zudem eine Anzahl innerer ringförmiger Fluidleitungsnuten (214) aufweist, die die inneren Dichtflächenabschnitte trennen;

b) einen Steuerschieber (218), der beweglich und abdichtend innerhalb der Ventilsitzhülse angeordnet ist und äußere beabstandete ringförmige Fluidleitungsnuten bestimmt, die selektiv mit den inneren ringförmigen Fluidleitungsnuten der Ventilsitzhülse verbunden sind und innere Fluidleitungsdurchgänge aufweisen; und

c) eine Vorrichtung (328), die ausgewählt den Steuerschieber hydraulisch aus der ersten Strömungssteuerstellung in die zweite Strömungssteuerstellung schiebt.

40. Einrichtung nach Anspruch 39, wobei die Einrichtung zumindest einen Probenspeichertank (320) enthält, der innerhalb des Gerätekörpers angeordnet ist, und der Steuerschieber enthält:

erste und zweite von einem Rückschlagventil gesteuerte Durchgänge (343, 345), die jeweils eine Formationsfluidströmung in eine Richtung gestatten und abhängig von der Stellung des Steuerschiebers ausgewählt betreibbar sind, um eine gepumpte Strömung des Formationsfluids von der oder zu der unterirdischen Bodenformation und zum oder vom Bohrloch oder Speichertank zu gestatten.

41. Einrichtung nach Anspruch 39, umfassend:

eine Auswahlvorrichtung (170) innerhalb des Gerätekörpers, die die gepumpte Formationsfluidströmung durch das Strömungsumkehrventil zum Bohrloch oder zum Probenspeichertank leitet.

42. Einrichtung nach Anspruch 41, wobei die Auswahlvorrichtung umfaßt:

a) einen Strömungsdurchgang (302), der die Durchgänge des Strömungsumkehrventils mit dem Bohrloch und dem Speichertank verbindet; und

b) zumindest ein Steuerventil (168) zum ausgewählten Steuern der Formationsfluidströmung aus dem Umkehrventil zum Bohrloch oder zum Probenspeichertank.

43. Gerät (13) für die Formationsprüfung im Bohrloch, umfassend:

a) einen Gerätekörper (32), eingerichtet zum Anordnen auf Formationstiefe innerhalb eines Bohrlochs, der eine Pumpkammer (36) und einen Fluidproben- und Pumpdurchgang (106) bestimmt, der mit der Pumpkammer verbunden ist;

b) einen Probenentnehmer (18), der gemeinsam mit der Gerätekörperanordnung angeordnet ist und seitlich daraus ausfahrbar ist, um für eine Fluidprobe in eine ausgewählte unterirdische Bodenformation einzugreifen, wobei der Probenentnehmer einen Fluidprobendurchgang (106) bestimmt, der fossiles Fluid aus der unterirdischen Bodenformation aufnimmt;

c) eine Dichtungsvorrichtung (25), die den Probendurchgang gegen den Bohrlochdruck isoliert und den Übergang des fossilen Fluids in den Probenentnehmer im wesentlichen auf Formationsdruck sicherstellt;

d) einen zweiseitigen Kolbenpumpenmechanismus (36), der innerhalb der Gerätekörperanordnung untergebracht ist und zumindest eine Pumpkammer (80) bestimmt, die in Fluidverbindung mit dem Fluidprobendurchgang steht, wobei die Pumpkammer weiterhin eine Prüfkammer für das fossile Fluid bestimmt, und der Kolbenpumpenmechanismus einen Kolben (86) hat, der sich in der Pumpkammer Hin- und Her bewegen kann, und der Kolben eine bewegliche Wand der Prüfkammer bestimmt;

e) eine Vorrichtung (116), die die Kolbenbewegung und -stellung innerhalb der Pumpkammer erfaßt und damit das endliche Volumen und die Volumenänderung der Prüfkammer und des darin enthaltenen fossilen Fluids erfaßt;

f) eine Vorrichtung (320), die ein endliches Volumen von fossilem Fluid innerhalb der Prüfkammer einschließt;

g) daß der Kolben (86) innerhalb der Pumpkammer ausgewählt beweglich ist, um das endliche Volumen von fossilem Fluid, das in der Prüfkammer enthalten ist, zum Bestimmen des Blasenpunktdrucks des Fluids zu verändern;

h) eine Vorrichtung (432) zum Erfassen der Temperatur des fossilen Fluids in der Prüfkammer; und

i) eine Vorrichtung zum Erfassen des anfänglichen Drucks (430) des fossilen Fluids in der Prüfkammer und zum Erfassen der Druckänderung des fossilen Fluids während der Volumenänderung des Fluids.

44. Gerät für die Formationsprüfung im Bohrloch nach Anspruch 43, umfassend:

ein Potentiometer innerhalb des Gerätekörpers, das so angeordnet ist, daß es die Kolbenbewegungen erfaßt, wobei das Potentiometer elektronische Positionsanzeigesignale liefert, die das begrenzte Volumen des fossilen Fluids innerhalb der Prüfkammer und eine Veränderung des begrenzten Volumens des fossilen Fluids innerhalb der Prüfkammer widerspiegeln.

45. Gerät für die Formationsprüfung im Bohrloch nach Anspruch 43, umfassend:

a) einen Fluiddurchgang (106), der durch den Gerätekörper bestimmt und mit dem Fluidprobendurchgang des Probenentnehmers und mit der Pumpkammer verbunden ist; und

b) eine Isoliervorrichtung (25), die verbunden mit dem Fluidprobendurchgang angeordnet ist und ausgewählt betrieben wird, um das begrenzte Volumen des fossilen Fluids innerhalb der Prüfkammer für eine Prüfung des Blasenpunktdrucks des Fluids festzuhalten.

46. Gerät für die Formationsprüfung im Bohrloch nach Anspruch 43, umfassend:

a) einen Temperatursensor (432), der mit der Pumpkammer (104) verbunden ist und die Formationstemperatur des fossilen Fluids innerhalb der Pumpenkammer erfaßt; und

b) einen Drucksensor (430), der mit der Pumpkammer (104) verbunden ist und den Formationsdruck des fossilen Fluids in der Pumpkammer und die Druckänderungen des begrenzten Volumens des fossilen Fluids während der Volumenänderung des Fluids erfaßt.

47. Gerät für die Formationsprüfung im Bohrloch nach Anspruch 43, umfassend:

a) eine bewegliche Wand (86), die einen Abschnitt der Prüfkammer bestimmt;

b) ein Potentiometer (116), das von der beweglichen Wand funktional betätigt werden kann und elektronische Ausgangssignale liefert, die die Stellungen der beweglichen Wand widerspiegeln und damit das Volumen der Prüfkammer, um das begrenzte Volumen und die Volumenänderung des fossilen Fluids innerhalb der Prüfkammer (104) zu bestimmen.

## Revendications

1. Procédé destiné à tester une formation souterraine (11) ayant un forage (10) défini dedans et contenant un fluide de formation, comportant :

   (a) le positionnement dans ledit forage d'un instrument de test de formation (13) ayant une sonde de prélèvement (18) et ayant une pompe à piston bidirectionnelle (36) qui est en communication de pompage avec ladite sonde de prélèvement pour le pompage sélectif dans et de ladite formation souterraine et ayant au moins un réservoir interne d'échantillon (320);
   (b) la projection de ladite sonde de prélèvement dudit instrument de test de formation en communication de prélèvement avec ledit forage au niveau de ladite formation souterraine;
   (c) avec ladite pompe à piston bidirectionnelle, le fait de forcer une quantité de fluide de test à travers ladite sonde de prélèvement et dans ladite formation souterraine; et
   (d) avec ladite pompe à piston bidirectionnelle, l'extraction d'une quantité dudit fluide de formation de ladite formation souterraine à travers ladite sonde de prélèvement et dans ledit instrument de test de formation.

2. Procédé selon la revendication 1, dans lequel des circuits de traitement des signaux sont prévus pour traiter des signaux électroniques de test de fluide, ledit procédé comportant en outre :

   (a) la réalisation d'au moins un essai de fluide de formation sur ledit fluide de formation dans ledit instrument de test et le développement de signaux électroniques représentant les résultats dudit essai de fluide de formation; et
   (b) la transmission desdits signaux électroniques dudit instrument de test de formation vers ledit circuit de traitement de signal.

3. Procédé selon la revendication 1, dans lequel des circuits de fluide ayant des soupapes pour la commande de ceux-ci sont situés dans ledit instrument de test de formation, ledit procédé comportant en outre :

   après ladite extraction, l'acheminement sélectif dudit fluide de formation grâce à des premiers réglages de soupape de ladite formation souterraine dans ledit réservoir d'échantillon, et grâce à des deuxièmes réglages de soupape, l'acheminement du fluide de ladite formation souterraine à travers lesdits circuits de fluide dudit instrument de test de formation dans ledit forage.

4. Procédé selon la revendication 1, dans lequel le fait de forcer ladite quantité de fluide de test à travers ladite sonde d'échantillon et dans ladite formation souterraine comporte :

   (a) avec ladite pompe à piston bidirectionnelle établissant une direction de pompage prédéterminée, le pompage de ladite quantité de fluide de test dans ladite formation souterraine à l'aide de ladite pompe à piston bidirectionnelle afin de se mélanger avec ledit fluide de formation de façon à définir un fluide de formation traité par du fluide de test de formation;
   (b) l'inversion de la direction de pompage de ladite pompe à piston bidirectionnelle par rapport à ladite direction de pompage prédéterminée; et
   (c) le pompage dudit fluide de formation traité par fluide de test de formation de ladite formation terrestre dans ledit instrument de test à l'aide de ladite pompe à piston bidirectionnelle dans ladite direction de pompage inversée de celle-ci.

5. Procédé selon la revendication 1, dans lequel ladite pompe à piston bidirectionnelle définit au moins une chambre de pompage (102), ledit procédé comportant en outre :

   le fait de forcer ledit fluide de test de formation qui est réalisé en pompant ledit fluide de test de formation de ladite chambre de pompage de ladite pompe à piston bidirectionnelle dudit instrument de test dans ladite formation souterraine.

6. Procédé selon la revendication 1, comprenant :

   le fait de forcer ledit fluide de test en pompant ledit fluide de test dudit forage à travers ledit instrurnent de test de formation et dans ladite formation souterraine.

7. Procédé selon la revendication 1, dans lequel :

   (a) le fait de forcer ladite quantité de fluide de l'étape (1) comporte l'établissement de la commande directionnelle de pompe de ladite pompe à piston bidirectionnelle par le positionnement sélectif du système de soupape d'instrument;
   (b) le pompage du fluide dudit instrument de test de formation à travers ladite sonde de prélèvement et dans ladite formation souterraine; et
   (c) l'inversion de la commande directionnellc de pompe de ladite pompe à piston bidirectionnellc par le repositionnement sélectif dudit système de soupape d'instrument; et

(d) ladite extraction de l'étape (d) comportant le pompage du fluide de forrnation de ladite formation souterraine à travers ladite sonde de prélèvement et dans ledit instrument de test de formation par une opération de pompage de ladite pompe à piston bidirectionnelle.

8. Procédé selon la revendication 7, dans lequel :

(a) après l'étape (d) de la revendication 7, on provoque le positionnement sélectif du système de soupape d'instrument; et
(b) on pompe le fluide de formation à travers ledit instrument de test de formation jusqu'audit forage afin de chasser lesdits débris de ladite formation souterraine.

9. Procédé selon la revendication 7, dans lequel ledit instrument de test de formation possède un réservoir de fluide (320) contenant un fluide de test de formation, ledit procédé comprenant :

(a) après l'étape (d) de la revendication 7, le fait de provoquer le positionnement sélectif du système de soupape d'instrument; et
(b) avec ladite pompe à piston bidirectionnelle (36), le pompage dudit fluide de test de formation dudit réservoir de fluide à travers ladite sonde de prélèvement afin de chasser lesdits débris de ladite formation souterraine.

10. Procédé selon la revendication 1, dans lequel ladite pompe à piston bidirectionnelle dudit instrument de test de formation comporte une pompe à piston à double effet et bidirectionnelle (36), ledit procédé comportant en outre :

(a) avec la pompe à piston bidirectionnelle à double effet, l'obtention d'un échantillon dudit fluide de formation;
(b) le changement de la pression dudit échantillon dudit fluide de formation à l'aide de ladite pompe à piston à double effet et bidirectionnelle; et
(c) la réalisation d'essais de pression, de volume et de température sur ledit échantillon à pression modifiée dudit fluide de formation.

11. Procédé selon la revendication 10, comportant en outre :

la répétition desdits essais de pression, de volume et de température jusqu'à ce qu'un échantillon de fluide choisi ait été obtenu.

12. Procédé selon la revendication 1, dans lequel ladite pompe à piston bidirectionnelle comporte un mécanisme de pompe à piston à double effet et bidirectionnelle qui est interconnecté avec ledit instrument de test de formation, ledit procédé comprenant :

l'actionnement de manière commandée dudit mécanisme de pompe à piston dans une direction de pompage choisie pour ladite étape (c) et l'actionnement de manière commandée dudit mécanisme de pompe à piston dans la direction de pompage choisie opposée pour ladite étape d'extraction (d).

13. Procédé selon la revendication 12, dans lequel, ledit mécanisme de pompe à piston bidirectionnelle ayant un corps de pompe (38) définissant une paire de chambres de pompage (102, 104) et ayant une chambre de piston d'actionnement (80) disposée entre lesdites chambres de pompage et ayant en outre un piston d'actionnement (86) qui est disposé pour un déplacement alternatif dans ladite chambre de piston d'actionnement (80) et ayant des pistons de pompage (94, 96) qui sont fixés sur et s'étendent de chaque côté dudit piston d'actionnement et qui sont reçus de manière respective pour un déplacement alternatif de pompage dans lesdites chambres de pompage, ledit procédé comportant en outre :

(a) avec ledit mécanisme de pompe à piston réglé dans une direction de pompage choisie, le déplacement alternatif de manière commandée desdits pistons d'actionnement et desdits pistons de pompage;
(b) la détection de la position d'au moins un desdits pistons d'actionnement et de pompage dans ledit corps de la pompe;
(c) la génération de signaux électroniques de position représentant ladite position détectée; et
(d) la corrélation desdits signaux électroniques de position avec le temps de déplacement du piston afin d'identifier le volume de fluide de formation pompé par ledit mécanisme de pompe à piston.

14. Procédé selon la revendication 13, comprenant :

la modification de manière électronique dudit vulumc de fluide de formation pompé par ledit mécanisme de pompe à piston.

15. Procédé selon la revendication 13, comprenant :

(a) la détection de la température du fluide de formation qui est extrait de la formation souterraine; et
(b) la corrélation de ladite température détectée de fluide de fonnation avec ladite cadence de pompage volumétrique afin d'établir le volume de précision corrigé en température de fluide de formation pompé par ledit mécanisme de pompe à piston.

16. Procédé selon la revendication 1, comprenant :

(a) la détection de la température du fluide de formation qui est extrait de ladite formation souterraine;
(b) la saisie d'un volume choisi dudit fluide de formation dans ledit instrument de test de formation; et
(c) la corrélation dudit volume fini dudit fluide de formation avec ladite température détectée de fluide de formation pour la détermination de précision du volume corrigé en température de celui-ci.

17. Procédé selon la revendication 16, dans lequel ledit fluide de formation a une pression de point de bulle, ledit procédé comportant en outre :

(a) avec ladite pompe à piston bidirectionnelle, le changement de manière commandée du volume dudit volume choisi de fluide de formation;
(b) l'observation de la pression dudit volume choisi de fluide de formation pendant ledit changement de volume de celui-ci afin de déterminer la pression de point de bulle dudit fluide de formation;
(c) le calcul de la compressibilité dudit fluide de formation; et
(d) l'utilisation de la pression de point de bulle et de la compressibilité dudit fluide de formation afin de déterminer la caractérisation de réservoir de ladite formation souterraine.

18. Procédé selon la revendication 1, dans lequel ledit fluide de formation a une pression de point de hulle et ladite pompe à piston bidirectionnelle (36) définit au moins une chambre de pompe (102) ayant un piston de pompe (94) dedans et dans laquelle ledit fluide de fonnation est conduit et de laquelle ledit fluide de formation est expulsé lors du mouvement linéaire du piston de pompe pendant l'activité de pompage, ladite chambre de pompe (102) étant en outre une chambre d'essai de pression de point de bulle et de compressibilité de fluide dudit instrument de test de formation, ledit instrument de test de formation ayant en outre des moyens (332, 330, 334, 116) destinés à détecter la température dudit fluide de formation, la pression dudit fluide de formation dans ladite chambre d'essai et le volume de ladite chambre d'essai, et ayant des moyens destinés à emprisonner ledit fluide de formation dans ladite chambre d'essai, ledit procédé comportant en outre :

(a) l'emprisonnement d'un volume fini dudit fluide de formation dans ladite chambre d'essai;
(b) la comparaison de la pression et du volume dudit fluide de formation retenu et la sortie de signaux électroniques représentatifs de ceux-ci,
(c) le changement de manière commandée du volume dudit fluide de formation retenu;
(d) l'observation des changements de pression dudit fluide de formation retenu pendant le changement de volume de celui-ci,
(e) la détermination de la pression de point de bulle dudit fluide de formation par comparaison de ladite pression, dudit volume, dudit changement de volume et dudit changement de pression du fluide de formation; et
(f) l'utilisation de ladite pression de point de bulle d'essais multiples de fluide de formation pour la caractérisation de réservoir de ladite formation souterraine.

19. Procédé d'élimination de débris d'une formation terrestre de substrat ayant un forage défini dedans et d'acquisition d'échantillons non contaminés de fluide de formation afin de tester celui-ci, ledit fluide de formation ayant une pression de point de bulle, ledit procédé comportant en outre:

(a) le positionnement dans ledit forage d'un instrument de test de forrnation (13) ayant une sonde de prélèvement (18) et ayant un réservoir de fluide intégré (320) et ayant une pompe à piston bidirectionnelle (36) en communication de pompage sélective avec ladite sonde de prélèvement et ledit réservoir de fluide embarqué;
(b) la projection de ladite sonde de prélèvement dudit instrument de test de formation en communication de prélèvement avec ladite formation souterraine;
(c) le pompage du fluide de formation de ladite formation souterraine à travers ledit instrument de test de formation et dans ledit forage jusqu'à ce que du fluide de formation non contaminé soit récupéré; et
(d) le pompage d'une quantité dudit fluide de formation non contaminé dans ledit réservoir de fluide intégré.

20. Procédé selon la revendication 19, comprenant :

(a) après ladite étape (c) de pompage de la revendication 19, la compression dudit fluide de formation à un niveau de pression qui dépasse la pression de point de bulle de celui-ci, et
(b) le maintien dudit niveau de pression de ladite formation afin d'empêcher une séparation de phase de celui-ci jusqu'à ce que l'analyse de point de bulle soit ensuite réalisée.

21. Procédé selon la revendication 19, comprenant :

    (a) la séparation de ladite sonde de prélèvement de ladite formation souterraine choisie une fois que ladite quantité dudit fluide de formation non contaminé a été acquise; et
    (b) la réalisation d'essais de fluide de formation avec ledit instrument de test de formation étant dans ledit forage et étant séparé de ladite formation souterraine choisie.

22. Procédé selon la revendication 21, comprenant :

    le déplacement dudit instrument de test de formation dans ledit forage tout en réalisant lesdits essais de fluide de formation.

23. Appareil pour la réalisation d'essais de pression, de volume et de température d'un échantillon de fluide fossile provenant d'une formation souterraine ayant un forage défini dedans et pour l'obtention d'échantillons de fluide fossile contenu dans ladite formation souterraine, comportant :

    (a) une structure de corps d'instrument (32) à positionner à une profondeur de formation choisie dans ledit forage et ayant un réservoir de fluide (320) contenant un fluide de test;
    (b) une sonde d'échantillon (310) qui est latéralement extensible depuis ladite structure de corps d'instrument pour l'engagement de prélèvement de fluide avec ladite formation souterraine, ladite sonde d'échantillon définissant un passage de prélèvement de fluide (318) destiné à admettre ledit échantillon de fluide fossile provenant de ladite formation souterraine dans ledit instrument et destiné à transférer du fluide dudit instrument vers ladite formation souterraine;
    (c) une pompe à piston bidirectionnelle (36) qui est disposée dans ladite structure de corps d'instrument et ayant un cylindre de pompe (80) et un piston (86) qui est mobile dans ledit cylindre de pompe; et
    (d) des moyens (350, 352, 360, 362) qui commandent de manière sélective la direction de pompage dudit mécanisme de pompe à piston pour le pompage sélectif dudit fluide de test à travers ladite sonde de prélèvement dans ladite formation terrestre et le pompage du fluide de formation de ladite formation souterraine à travers ladite sonde de prélèvement et dans ladite structure de corps.

24. Appareil selon la revendication 23, dans lequel :

    (a) ledit corps d'instrument définit une chambre de test (102) prévue pour contenir un volume fini dudit fluide fossile;
    (b) des moyens (330, 332) destinés à détecter la pression et la température de formation dudit fluide fossile;
    (c) des moyens (116) destinés à modifier le volume et à détecter le changement de volume dudit volume fini dudit fluide fossile dans ladite chambre de test; et
    (d) des moyens (324) destinés à détecter des changements de pression dudit volume fini dudit fluide fossile pendant ledit changement du volume de celui-ci afin d'établir le point de pression de bulle dudit fluide fossile.

25. Appareil selon la revendication 24, comprenant :

    des moyens (116, 324) associés à ladite chambre de test afin de déterminer la compressibilité dudit fluide fossile dans ladite chambre de test.

26. Appareil selon la revendication 24, dans lequel :

    ledit mécanisme de pompe à piston définit au moins un mécanisme de chambre de pompe (80) ayant un piston (86) alternativement mobile dedans pour une activité de pompage, ladite chambre de pompe définissant ladite chambre de test (104).

27. Appareil selon la revendication 26, dans lequel ledit piston (86) définit lesdits moyens de changement du volume dudit volume fini dudit fluide fossile.

28. Appareil selon la revendication 27, comprenant :

    un potentiomètre linéaire (116) dans ledit corps d'instrument (32) et qui est disposé en relation de détection de mouvement avec ledit piston, ledit potentiomètre linéaire fournissant des signaux électroniques d'indication de position qui reflète ledit volume fini dudit fluide fossile dans ladite chambre de test et le changement dudit volume fini dudit fluide fossile dans ladite chambre de test.

29. Appareil selon la revendication 26, comprenant :

    (a) un passage de fluide (426) défini par ledit corps d'instrument et qui est en communication avec ledit passage de prélèvement de fluide de ladite sonde de prélèvement et avec ladite chambre de pompe (102); et
    (b) des moyens d'isolation (428) qui sont disposés en communication avec ledit passage de prélèvement de fluide et qui sont actionnés de manière sélective afin de piéger ledit volume fini dudit fluide fossile dans ladite chambre d'essai pour des essais de pression de point de bulle de celui-ci.

**30.** Appareil selon la revendication 26, comprenant :

(a) un capteur de température (432) qui est relié à ladite chambre de pompe (102) afin de détecter la température de formation dudit fluide fossile dans ladite chambre de test; et
(b) un capteur de pression (430) qui est relié à ladite chambre de pompe (102) afin de détecter la pression de formation dudit fluide fossile dans ladite chambre de pompe (102) et afin de détecter lesdits changements de pression dudit volume fini dudit fluide fossile pendant ledit changement de volume de celui-ci.

**31.** Appareil selon la revendication 24, comprenant :

(a) une paroi mobile (86) définissant une partie de ladite chambre de test; et
(b) un potentiomètre (116) qui peut être déplacé par ladite paroi mobile et qui délivre des signaux de sortie électroniques reflétant des positions de ladite paroi mobile et ainsi du volume de ladite chambre de test pour la détermination dudit volume fini et du changement de volume dudit fluide fossile dans ladite chambre de test.

**32.** Appareil selon la revendication 23, comportant en outre :

(a) des moyens de détection de position (116) destinés à détecter la position de ladite pompe à piston bidirectionnelle (36) et à générer des signaux électroniques de position de piston; et
(b) des moyens (22) destinés à recevoir et traiter lesdits signaux électroniques de position de piston pour l'identification du volume de fluide fossile pompé par ladite pompe à piston bidirectionnelle.

**33.** Appareil selon la revendication 32, comportant en outre :

(a) des moyens (330, 332) destinés à détecter la pression et la température du fluide de formation qui entre dans ledit instrument de test de formation en provenance de ladite formation souterraine et à générer des signaux électroniques de pression et de la température qui s'y rapportent; et
(b) des moyens (22) destinés à traiter lesdits signaux électroniques de pression et de température en corrélation avec lesdits signaux électroniques de position de piston pour l'obtention du déplacement volumétrique corrigé en pression et en température dudit fluide fossile par ledit mécanisme de pompe à piston.

**34.** Appareil selon la revendication 23, dans lequel :

(a) ledit mécanisme de pompe à piston bidirectionnelle (19) est une pompe à piston bidirectionnelle (36) ayant des chambres de pompage opposées (102, 104);
(b) lesdits moyens (168, 170) destinés à commander de manière sélective la direction de pompage de ladite pompe à piston étant un circuit de pompage de fluide hydraulique ayant des clapets anti-retour permettant l'écoulement unidirectionnel du fluide vers et depuis lesdites chambres de pompage; et
(c) une soupape d'inversion (240, 242) reliée audit circuit de pompage de fluide hydraulique afin de sélectionner la direction d'écoulement de fluide vers et depuis lesdits clapets anti-retour.

**35.** Appareil selon la revendication 32, dans lequel ladite pompe à piston bidirectionnelle définit une paire de cylindres de pompe ayant chacun un piston de pompage disposé dedans de façon mobile et lesdits moyens de détection de position (116) comportent :

(a) une tige d'indicateur de position (114) s'étendant depuis un desdits pistons de pompage; et
(b) un potentiomètre à déplacement linéaire qui reçoit ladite tige d'indicateur de position et qui génère lesdits signaux électroniques de position de piston reflétant la position de ladite tige d'indicateur de position par rapport audit potentiomètre à déplacement linéaire.

**36.** Appareil selon la revendication 23, dans lequel ledit mécanisme de pompe à piston bidirectionnelle comporte :

(a) un corps (36) de la pompe définissant une paire de chambres de pompage (102, 104) et une chambre de piston d'actionnement (80) disposée entre lesdites chambres de pompage;
(b) un piston d'actionnement (86) disposé pour un déplacement alternatif activé de manière hydraulique dans ladite chambre de piston d'actionnement (80);
(c) une paire de pistons de pompage (94, 96) fixés sur et s'étendant depuis des côtés opposés dudit piston d'actionnement (86) et reçus pour un déplacement alternatif de pompage dans les chambres de pompage respectives (102, 104);

(d) un circuit d'actionnement de pompe hydraulique (figure 5) destiné à induire le déplacement alternatif d'actionnement dudit piston d'actionnement dans ladite chambre de piston d'actionnement;

(e) un circuit de fluide fossile (figure 6) qui est disposé dans ladite structure de corps d'instrument et qui est en communication avec lesdites chambres de pompage et recevant du fluide de formation provenant desdites chambres de pompage lors du déplacement alternatif desdits pistons de pompage; et

(f) des moyens de commande directionnelle (figure 4) destinés à diriger de manière sélective l'écoulement du fluide de formation dans ledit circuit de fluide de formation vers ladite formation souterraine ou depuis ladite formation souterraine.

37. Appareil selon la revendication 36, dans lequel ledit circuit de fluide de formation (figure 6) comprend une source de fluide dans ledit corps d'instrument et un réceptacle de fluide dans ledit corps d'instrument et lesdits moyens de commande directionnelle comportent :

(a) une soupape d'inversion d'écoulement (168) dans ledit circuit de fluide de formation ayant une première position de commande d'écoulement qui dirige l'écoulement de fluide de formation de ladite formation souterraine vers lesdites chambres de pompage (102, 104) et desdites chambres de pompage vers un récepteur d'échantillon (320), ladite soupape d'inversion d'écoulement ayant une deuxième position de commande d'écoulement qui dirige le fluide de ladite source de fluide vers lesdites chambres de pompage et desdites chambres de pompage vers ladite formation souterraine; et

(b) des moyens (240) destinés à positionner de manière commandée ladite soupape d'inversion d'écoulement dans ladite première ou ladite deuxième position de commande d'écoulement.

38. Appareil selon la revendication 37, dans lequel lesdits moyens destinés à positionner de manière commandée ladite soupape d'inversion d'écoulement comportent :

(a) une alimentation en pression pilote (236) qui est reliée de manière opérationnelle à ladite soupape d'inversion d'écoulement; et

(b) une soupape de commande (240) qui commande de manière sélective l'application de la pression pilote de ladite alimentation en pression pilote sur ladite soupape d'inversion pour l'actionnement sélectionné de celle-ci dans lesdites première et deuxième positions de commande d'écoulement.

39. Appareil selon la revendication 37, dans lequel ladite soupape d'inversion d'écoulement (168) comporte :

(a) une douille de siège de soupape (192) qui est retenue en relation étanche dans lesdites structures de corps d'instrument et qui forme une pluralité de segments internes de surface d'étanchéité, ladite douille de siège de soupape ayant des rainures de conduite de fluide externes annulaires espacées disposées en communication avec des passages respectifs d'écoulement de fluide hydraulique et de fluide de formation de ladite structure de corps d'instrument, ladite douille de siège de soupape ayant en outre une pluralité des rainures de conduite de fluide annulaires internes (214) qui séparent lesdits segments internes de surface d'étanchéité;

(b) un tiroir de soupape (218) qui est positionné de façon mobile en relation étanche dans ladite douille de siège de soupape et qui définit des rainures de conduite de fluide annulaires externes espacées en communication sélective avec lesdites rainures de conduite de fluide annulaires internes de ladite douille de siège de soupape et ayant des passages de conduite de fluide internes; et

(c) des moyens (328) qui déplacent de manière hydraulique et sélective ledit tiroir de soupape de ladite première position de commande d'écoulement vers ladite deuxième position de commande d'écoulement.

40. Appareil selon la revendication 39, dans lequel ledit appareil comprend au moins un réservoir de stockage d'échantillon (320) disposé dans ledit corps d'instrument et ledit tiroir de soupape comprend :

des premier et deuxième passages commandés par clapet anti-retour (343, 345) permettant chacun un écoulement unidirectionnel de fluide de formation et pouvant être actionnés de manière sélective en fonction de la position dudit tiroir de soupape afin de permettre un écoulement pompé de fluide de formation depuis ou vers ladite formation souterraine et depuis ou vers ledit forage ou réservoir de stockage d'échantillon.

41. Appareil selon la revendication 39, comprenant :

des moyens de sélection (170) dans ledit corps d'instrument destinés à diriger l'écoulement pompé de fluide de formation à travers ladite soupape d'inversion d'écoulement vers ledit forage ou vers ledit réservoir de stockage d'échantillon.

42. Appareil selon la revendication 41, dans lequel lesdits moyens de sélection comportent :

(a) un passage d'écoulement (302) reliant lesdits passages de soupape d'inversion d'écoulement audit forage et audit réservoir de stockage; et
(b) au moins une soupape de commande (168) destinée à commander de manière sélective l'écoulement du fluide de formation de ladite soupape d'inversion vers ledit forage ou vers ledit réservoir de stockage d'échantillon.

43. Instrument de test de formation de fond de trou (13) comportant :

(a) un corps d'instrument (32) prévu pour un positionnement à une profondeur de formation dans un forage et définissant une chambre de pompe (36), un passage de prélèvement et de pompage de fluide (106) en communication avec ladite chambre de pompe;
(b) une sonde d'échantillon (18) prévue en coopération sur et latéralement extensible depuis ladite structure de corps d'instrument pour l'engagement de prélèvement de fluide avec une formation souterraine choisie, ladite sonde d'échantillon définissant un passage d'échantillon de fluide (106) destiné à admettre le fluide fossile de ladite formation souterraine;
(c) des moyens d'obturateur (25) qui isolent ledit passage de prélèvement de la pression de forage et assurent la communication du fluide fossile dans ladite sonde d'échantillon sensiblement à la pression de formation;
(d) un mécanisme de pompe à piston bidirectionnelle (36) qui est disposé dans ladite structure de corps d'instrument et définissant au moins une chambre de pompage (80) ayant une communication de fluide avec ledit passage de prélèvement de fluide, ladite chambre de pompage définissant en outre une chambre de test pour ledit fluide fossile, ledit mécanisme de pompage à piston ayant un piston (86) alternativement mobile dans ladite chambre de pompage, ledit piston définissant une paroi mobile de ladite chambre de test;
(e) des moyens (116) destinés à détecter le mouvement de piston et le positionnement dans ladite chambre de pompage et à détecter ainsi le volume fini et le changement de volume de ladite chambre de test et du fluide fossile contenu dedans;

(f) des moyens (320) destinés à piéger un volume fini de fluide fossile dans ladite chambre de test;
(g) ledit piston (86) étant mobile de manière sélective dans ladite chambre de pompage afin de modifier ledit volume fini de fluide fossile contenu dans ladite chambre de test pour la détermination de la pression de point de bulle de celui-ci,
(h) des moyens (432) destinés à détecter la température du fluide fossile dans ladite chambre de test; et
(i) des moyens destinés à détecter la pression initiale (430) du fluide fossile dans ladite chambre de test et à détecter le changement de pression dudit fluide fossile pendant le changement de volume de celui-ci.

44. Instrument de test de formation de fond de trou selon la revendication 43, comprenant :

un potentiomètre dans ledit corps d'instrument qui est disposé en relation de détection de mouvement avec ledit piston, ledit potentiomètre délivrant des signaux électroniques d'indication de position reflétant ledit volume fini dudit fluide fossile dans ladite chambre de test et le changement dudit volume fini dudit fluide fossile dans ladite chambre de test.

45. Instrument de test de formation de fond de trou selon la revendication 43, comprenant :

(a) un passage de fluide (106) défini par ledit corps d'instrument et qui est en communication avec ledit passage de prélèvement de fluide de ladite sonde de prélèvement et avec ladite chambre de pompe; et
(b) des moyens d'isolation (25) qui sont disposés en communication avec ledit passage de prélèvement de fluide et qui sont actionnés de manière sélective afin de piéger ledit volume fini dudit fluide fossile dans ladite chambre d'essai pour des essais de pression de point de bulle de celui-ci.

46. Instrument de test de formation de fond de trou selon la revendication 43, comprenant :

(a) un capteur de température (432) qui est relié à ladite chambre de pompe (104) afin de détecter la température de formation dudit fluide fossile dans ladite chambre de test; et
(b) un capteur de pression (430) qui est relié à ladite chambre de pompe (104) afin de détecter la pression de formation dudit fluide fossile dans ladite chambre de pompe et afin de détecter lesdits changements de pression dudit volume fini dudit fluide fossile pendant ledit changement de volume de celui-ci.

47. Instrument de test de formation de fond de trou selon la revendication 43, comprenant :

(a) une paroi mobile (86) définissant une partie de ladite chambre de test;
(b) un potentiomètre (116) qui est déplacé de manière opérationnelle par ladite paroi mobile et qui délivre des signaux électroniques de sortie reflétant des positions de ladite paroi mobile et ainsi le volume de ladite chambre de test pour la détermination dudit volume fini et du changement de volume dudit fluide fossile dans ladite chambre de test (104),

EP 0 646 215 B1

FIG.1

FIG.2

FIG.3

*FIG.4B*

PUMP THROUGH
PISTON

BOREHOLE
OR TANK

PUMP
PRESSURE

FLOW

HYDRAULIC
RESERVOIR

FIG. 4A

PUMP THROUGH
PISTON

BOREHOLE
OR TANK

PUMP
PRESSURE

FLOW

HYDRAULIC
RESERVOIR

FIG.5

PACKER

BOREHOLE
OR
TANK

FIG.6

TANK OR
BORHOLE
LINE

PACKER LINE
(FORMATION)

FIG.7

*FIG.8*

*FIG.9*

FIG.11

FIG.10